# EUROPEAN PATENT SPECIFICATION

(11) **EP 3 622 041 B1**
(45) Date of publication and mention of the grant of the patent: **01.07.2026**
(21) Application number: 17734151.8
(22) Date of filing: 12.05.2017
(51) Int. Cl.: C10K 1/16, B01D 53/14

(54) **REMOVAL OF MONOCYCLIC AROMATIC COMPOUNDS (BTEX) FROM A GAS**
ENTFERNUNG VON MONOCYCLISCHEN AROMATISCHEN VERBINDUNGEN (BTEX) AUS EINEM GAS
ÉLIMINATION DE COMPOSÉS AROMATIQUES MONOCYCLIQUES (BTEX) CONTENUS DANS UN GAZ

(43) Date of publication of application: 18.03.2020
(73) Proprietor: Nederlandse Organisatie voor toegepast- natuurwetenschappelijk Onderzoek TNO, 2595 DA Den Haag (NL)
(72) Inventor: VREUGDENHIL, Berend Joost, 2595 DA, 's-Gravenhage (NL); BOS, Alexander, 2595 DA, 's-Gravenhage (NL)
(74) Representative: Nederlandsch Octrooibureau
(86) International application number: PCT/NL2017/050297
(87) International publication number: WO 2018/208144

(56) References cited:
- EP-A2- 0 221 571
- WO-A1-2014/051419
- US-A- 3 877 893
- US-A- 4 025 322
- US-A- 4 046 641

## Description

The present invention relates to an improved process for purifying a gas by removal of aromatic compounds.

### Background

An energy gas is a gas that comprises gaseous fuels such as H₂, CO, CH₄ and other lower hydrocarbons (e.g. C₁ - C₄ alkanes and C₁ - C₄ alkenes). Such gases typically originate from gasification or pyrolysis from solid fuels, such as coal, biomass or waste and may be used in the production of heat, electricity, Fischer-Tropsch Diesel, substitute natural gas (SNG) and the like. The use of biomass and waste as feedstock is attractive for producing renewable products and energy, such as bio-SNG. Reducing the society's dependence on fossil fuels is currently a major issue.

Gases obtained by gasification or pyrolysis typically contain gaseous fuels, such as H₂, CO₂, CO and lower hydrocarbons, together with further organic compounds such as monocyclic aromatic compounds as well as tar-like polycyclic aromatic components. Especially gasification or pyrolysis of biomass and waste yields aromatic compounds. In biomass, large structures of aromatic moieties (e.g. lignin) are typically present. To be able to use the energy gas in the applications mentioned above, especially the tars and further aromatic compounds need to be removed from the gas. Purifying gases such as energy gases from tars is typically accomplished by contacting the (energy) gas with a washing liquid, during which tars are removed from the gas and absorbed in the washing liquid, which may then be stripped to remove the tars from the washing liquid (see Rabou et al., Energy Fuels, 2009, 23, 6189-6198). Conventional process for purifying gases however do not sufficiently remove the lowest, i.e. monocyclic, aromatic compounds, such as benzene, toluene, ethylbenzene and xylene, from the gas, which thus needs to be further treated to convert these compounds to gaseous fuels such as H₂, CO₂, CO and CH₄.

WO 03/018723 describes a process for removing tars with an aromatic hydrocarbon based washing liquid from a synthesis gas obtained during gasification of biomass. Gasification gas is subjected to a two-step cleaning treatment for removing tars. In a first step the gas is condensed in a washing liquid which is a hydrocarbon oil, wherein heavy tars (such as C₁₈ and higher) are removed. In an optional second washing stage, remaining tars (typically a mixture containing benzene, toluene, naphthalene, phenol and the like) in an absorption column. A further process using hydrocarbon oil as washing liquid is known from WO 2008/010717.

US 5772734 discloses the use of a wide variety of organic washing liquid, including lowviscosity silicone oils, light minerals oils and glycol ether especially triethylene glycol dibutyl ether for removing organic compounds such as chlorinated hydrocarbons or aromatic hydrocarbons from industrial gas streams. WO 2014/051419 describes a process for the purification of a gas stream, wherein the gas stream is contacted with a liquid organic polysiloxane at a temperature of 30 - 150 °C. Such contacting results in removal of tar-like components in a single step. Light aromatic compounds such a benzene are not sufficiently removed by the contacting step, and the benzene that is removed is obtained in a mixture with the tar-like components (heavy aromatic compounds). Separation of such mixtures of light and heavy aromatic compounds is not economically feasible, causing huge waste streams and loss of the fraction of light aromatic compounds such as benzene as a valuable product.

There remains a need in the art for a gas purification process that does not employ hydrocarbon-based washing liquids and at the same time provides efficient removal of aromatic compounds such as benzene in a separate fraction.

### Summary of the invention

The present invention relates to an improved process for purifying a gas as defined in claim 1.

The inventors surprisingly found that this process, in particular the use of steam in step (b) provides efficient separation of the aromatic compounds from the incoming gas. Moreover, also when step (a) is performed at reduced temperature, the process provides efficient removal of monocyclic aromatic compounds. As such, a valuable fraction of lower monocyclic aromatic compounds, such as benzene, toluene, ethylbenzene and xylene (together referred to as BTEX), is separated from the gas, which may be marketed as a separate product. At the same time a gas is provided that is substantially free of aromatic components, including the lightest ones such as benzene, toluene and even thiophene. It was found that removal of light and heavy tar-like components in a single contacting step gave significant "slip through" of particularly benzene and thiophene, which was avoided in the process according to the invention. This is particularly important when the incoming gas stream originates from a gasification or pyrolysis reactor, as such gases contain significant amounts of benzene. In prior art processes for purifying a gas, slipped through compounds remain in the gas stream and further measures are needed to remove them in order to be able to apply the gas in e.g. substitute natural gas (SNG) or electricity production.

Furthermore, the present invention eliminates the need for a steam reforming step when energy gases, e.g. originating from gasification or pyrolysis of biomass and/or coal, are converted in SNG. Any remaining (monocyclic) aromatic compounds such as benzene should be converted into syngas (by steam reforming) and subsequently into methane. The present process eliminates these two steps as benzene and related aromatic compounds are effectively removed from the gas stream. Since also thiophene is effectively removed, hydrodesulphurization (HDS) of the gas may be performed to a lower extent, e.g. only for the conversion of small sulphur-containing compounds into H₂S and subsequent removal thereof. Thiophene is generally known to be among the most challenging compounds to be removed by HDS, and the process according to the invention eliminates the need of thiophene removal by HDS, which is thus a marked improvement over prior art processes. Depending on the composition of the incoming gas stream, traces of monocyclic aromatic compounds and sulphur capture may be performed by contacting with activated carbon or may not even be needed altogether.

Furthermore, the present invention reduces the required efficiency of H₂S removal when energy gases, e.g. originating from gasification or pyrolysis of biomass, waste and/or coal are converted in electricity. Any remaining thiophenes are not easily captured from the product gas by conventional sulphur removal processes like amine or caustic scrubbers. The effective removal of thiophenes by the present invention eliminates the formation of SOₓ from thiophenes, therefore allowing for more SOₓ formed from H₂S and COS while remaining below the SOₓ emission limits. If conventional sulphur removal processes are required to reach SOₓ emission limits, a lower efficiency suffices on H₂S and COS removal in the absence of thiophenes. A further advantage is that conventional biological sulphur removal processes can be applied as the for these processes poisonous BTEX components are removed upstream the biological process.

A further major advantage is that biomass-derived BTEX (mixture of one or more of benzene, toluene, ethylbenzene and xylene) has now become available, in case the process according to the invention is used to purify a gas originating from biomass gasification or pyrolysis. The BTEX fraction is effectively separated from the gas stream and from the heavier tar-like components, using the process according to the invention. Benzene, toluene, ethylbenzene and xylene are valuable chemicals for the production of e.g. plastics (e.g. polystyrene), paints, coatings, resins and the like. The fraction of monocyclic aromatic compounds removed from the incoming gas in step (a) is an ideal starting material for benzenebased industries to develop biomass-based products and as such contribute to the overall reduction in CO₂ emission.

A further advantage of the process according to the invention is associated with the use of steam as stripping gas. Condensation of the stripping gas loaded with monocyclic aromatic compounds enables efficient isolation of the monocyclic aromatic compounds as a valuable product (BTEX). The inventors found that using steam as stripping gas enabled stripping and especially separating of the monocyclic aromatic compounds from the stripping gas at much more lenient conditions, compared to the use of permanent gases such as nitrogen or air as stripping gas. Using the latter, effective separation of the monocyclic aromatic compounds was only feasible at a temperature below 0 °C, such as -6 °C, at which temperature water, which is typically present in at least trace amounts during step (c), freezes, which hampers condensation and separation of the monocyclic aromatic compounds from the other components.

### Detailed description

The present invention relates to an improved process for purifying a gas containing monocyclic aromatic compounds.

### Process

The first aspect of the invention concerns a process according to claim 1.

The process according to the invention removes aromatic compounds from a gas, preferably an energy gas, thereby purifying the gas. The gas to be treated in the process according to the invention is also referred to as the "incoming gas" or just "the gas". The term "permanent gas" refers to those compounds that remain gaseous throughout the entire process.

In the process according to the invention, the incoming gas is in step (a) contacted with a washing liquid at a temperature of 15 - 60 °C, to obtain a purified gas and a spent washing liquid. The purified gas is depleted in aromatic compounds and is the first main product of the process according to the invention, while the spent washing liquid contains the aromatic compounds that are removed from the incoming gas. The spent washing liquid is subsequently stripped (i.e. the aromatic compounds are removed) in step (c), wherein the spent washing liquid is contacted with a stripping gas comprising stream to obtain a loaded stripping gas and a stripped washing liquid. The stripped washing liquid is conveniently reused as washing liquid in step (a). The loaded stripping gas obtained in step (b) contains (or "is loaded with") the aromatic compounds that are removed from the spent washing liquid. The process according to the invention further comprises a separation step (c), wherein the aromatic compounds are separated from the loaded stripping gas to obtain a cleared stripping gas. The separated monocyclic aromatic compounds are a second main product, which is obtained by the process according to the invention. The cleared stripping gas, from which the aromatic compounds have been removed, obtained in step (c), is conveniently reused in step (b).

In a first embodiment outside of the scope of the invention, the contacting of step (a) occurs at a temperature of 60 - 250 °C, and the aromatic compounds include one or more of phenol and polycyclic aromatic compounds, collectively referred to as "tars" or "tar-like components". Step (a) being performed at a temperature of 60 - 250 °C is also referred to as "detarring". The purified gas obtained in step (a) or (a") may also be referred to as "detarred gas". The detarred gas is depleted in tar-like components, while the spent washing liquid contains the tar-like components that are removed from the incoming gas. In the invention, the contacting of step (a) occurs at a temperature of 15 - 600 °C, and the aromatic compounds include monocyclic aromatic compounds, also referred to as "BTEX". Step (a) being performed at a temperature of 15 - 60 °C is also referred to as "BTEX scrubbing". The purified gas obtained in step (a) or (a') may also be referred to as "BTEX-depleted gas". The BTEX-depleted gas is depleted in monocyclic aromatic compounds, while the spent washing liquid contains the monocyclic aromatic compounds that are removed from the incoming gas. It is particularly preferred that the process according to the invention includes a detarring step (i.e. step (a")) and a BTEX scrubbing step (i.e. step (a) performed a temperature of 15 - 60 ºC)), wherein the BTEX scrubbing step is performed according to the invention, i.e. as defined by steps (a), (b) and (c). In one embodiment outside of the scope of the invention, at least the detarring step is performed according to the invention. The BTEX scrubbing step is performed according to the invention. Most preferably, the BTEX and the detarring step are performed according to the invention.

In the first embodiment, which falls outside of the scope of the invention, the contacting of step (a) occurs at a temperature of 60 - 250 °C, preferably 90 - 220 °C, more preferably 120 - 190 °C, and the aromatic compounds include tar-like components. In other words, the process according to the first embodiment comprises:
(a) contacting the gas with a washing liquid, at a temperature of 60 - 250 °C, to obtain the purified gas and a spent washing liquid comprising tar-like components;
(b) contacting the spent washing liquid with a stripping gas comprising at least 50 vol.% steam, to obtain a loaded stripping gas comprising tar-like components and a stripped washing liquid; and
(c) separating tar-like components from the loaded stripping gas obtained in step (b) by condensation of the steam and/or the tar-like components comprised in the loaded stripping gas to obtain an immiscible composition and isolating the tar-like components therefrom.

In the context of the first embodiment, it is preferred that the purified gas obtained in step (a) is further purified by:
(a') contacting the gas with a post-washing liquid, at a temperature of 15 - 60 °C, to obtain the further purified gas and a spent post-washing liquid comprising monocyclic aromatic compounds;
(b') contacting the spent post-washing liquid with a post-stripping gas, to obtain a loaded post-stripping gas comprising aromatic the monocyclic aromatic compounds and a stripped post-washing liquid; and
(c') optionally separating the monocyclic aromatic compounds from the loaded post-stripping gas.

Herein, it is preferred that step (a') is performed as defined for step (a) according to the second preferred embodiment. Likewise, it is preferred that step (b') is performed as defined for step (b) according to the second preferred embodiment. Further, it is preferred that step (c') is performed, and performed as defined for step (c) according to the second preferred embodiment.

In the second embodiment, which is according to the present invention, the contacting of step (a) occurs at a temperature of 15 - 60 °C, preferably 25 - 50 °C, more preferably 30 - 40 °C, and the aromatic compounds include monocyclic aromatic compounds. In other words, the process according to the first embodiment comprises:
(a) contacting the gas with a washing liquid, at a temperature of 15 - 60 °C, to obtain the purified gas and a spent washing liquid comprising monocyclic aromatic compounds;
(b) contacting the spent washing liquid with a stripping gas comprising at least 50 vol.% steam, to obtain a loaded stripping gas comprising monocyclic aromatic compounds and a stripped washing liquid; and
(c) separating monocyclic aromatic compounds from the loaded stripping gas obtained in step (b) by condensation of the steam and/or the monocyclic aromatic compounds comprised in the loaded stripping gas to obtain an immiscible composition and isolating the monocyclic compounds therefrom.

In the context of the second embodiment, it is preferred that the gas is subjected to a pre-washing step prior to being subjected to step (a), wherein the pre-washing comprises:
(a") contacting the gas with a pre-washing liquid, at a temperature of 60 - 250 °C, to obtain pre-purified gas which is subjected to step (a) and a spent pre-washing liquid comprising aromatic compounds including tar-like components;
(b") contacting the spent pre-washing liquid with a pre-stripping gas, to obtain a loaded pre-stripping gas comprising the tar-like components and a stripped pre-washing liquid; and
(c") optionally separating the tar-like components from the loaded pre-stripping gas.

Herein, it is preferred that step (a") is performed as defined for step (a) according to the first preferred embodiment. Likewise, it is preferred that step (b") is performed as defined for step (b) according to the first preferred embodiment. Further, it is preferred that step (c") is performed, and performed as defined for step (c) according to the first preferred embodiment.

In one embodiment, step (b) is performed directly following step (a), without any significant further alterations to the spent washing liquid obtained in step (a). In one embodiment, step (c) is performed directly following step (b), without any significant further alterations to the loaded stripping gas obtained in step (b). In one embodiment, step (a') is performed directly following step (a), without any significant further alterations to the detarred gas obtained in step (a). In one embodiment, step (b') is performed directly following step (a'), without any significant further alterations to the spent post-washing liquid obtained in step (a'). In one embodiment, step (c') is performed directly following step (b'), without any significant further alterations to the loaded post-stripping gas obtained in step (a'). In one embodiment, step (b") is performed directly following step (a"), without any significant further alterations to the spent pre-washing liquid obtained in step (a"). In one embodiment, step (c") is performed directly following step (b"), without any significant further alterations to the loaded pre-stripping gas obtained in step (a'). In one embodiment, step (a) is performed directly following step (a"), without any significant further alterations to the pre-purified gas obtained in step (a"). In one embodiment, the process according to the invention does not comprise a step wherein monocyclic aromatic compounds such as benzene are converted into syngas. In one embodiment, the process according to the invention does not comprise a step wherein tar-like components such as naphthalene are converted into syngas. In one embodiment, the process preferably does not comprise a steam reforming step. In one embodiment, the process according to the invention does not comprise a hydrodesulphurization step wherein thiophene is converted to H₂S, more preferably the process according to the invention does not comprise a hydrodesulphurization step at all.

The process according to the invention thus provides a purified gas, which is depleted in aromatic compounds and a fraction of aromatic compounds. The process according to the invention may provide a fraction of monocyclic aromatic compounds, a fraction of tar-like components, or both. All two or three products can separately be further used as deemed fit. The purified gas is preferably used as energy gas, e.g. for the production of electricity, or as (bio-)SNG, optionally after further cleaning or conversion steps as explained below. The fraction of monocyclic aromatic compounds is a valuable product, e.g. as starting material for benzenebased industries to develop biomass-based products. In a preferred embodiment, the fraction of tar-like components is recycled to the (biomass) gasification or pyrolysis reactor, from which the incoming gas preferably originates, and subjected again to gasification or pyrolysis, or used for heating of the reactor.

In the process according to the invention, the concentration of aromatic compounds in the gas to be purified decreases during step (a). Such reduction in concentration is also referred to as "depleted in". The gas depleted in aromatic compounds refers to a gas which comprises aromatic compounds in a reduced concentration (e.g. based on total volume of the gas) compared to the concentration of aromatic compounds in the incoming gas. Thus, the contacting of step (a) affords a gas which is depleted in aromatic compounds, which is also referred to as the "purified gas". During step (a) the concentration of aromatic compounds in the washing liquid increases, after which the washing liquid containing the aromatic compounds is referred to as "spent". In the process according to the invention, the concentration of aromatic compounds in the washing liquid decreases during respectively steps (b) and (b') or (b"), after which the washing liquid is referred to as "stripped", while at the same time the concentration of aromatic compounds and/or tar-like components increases in the stripping gas, which is then referred to as "loaded". In the context of the present invention, "loaded with" may also be referred to as "comprising". In step (c), the concentration of aromatic compounds in the stripping gas is decreased again, after which the stripping gas is referred to as "cleared".

The process according to the invention involves several steps which are executed in a certain order. In the context according to the invention, whenever a step 1 is performed after a step 2 refers to step 2 being upstream of step 1. Likewise, whenever a step 1 is performed before or prior to a step 2 refers to step 2 being downstream of step 1.

### Incoming gas

The gas to be purified can be any gas comprising small molecules such as hydrogen, nitrogen, small hydrocarbons (up to 4 carbon atoms, such as methane and ethane), carbon monoxide and carbon dioxide. Typically, the gas stream comprises at least one of hydrogen, CO and C₁₋₄ alkanes, preferably at least one of hydrogen, CO and methane. The gas may also contain larger alkanes (e.g. up to 6 or even 7 carbon atoms) and olefins. Furthermore, the incoming gas contains monocyclic aromatic compounds. For example, the gas may comprise hydrogen, carbon monoxide, carbon dioxide, methane, nitrogen, and water (vapour), in addition to the aromatic compounds. The gas is in particular an energy gas or a synthesis gas, typically containing one or more of hydrogen, carbon monoxide, and methane, in addition to varying levels of nitrogen, carbon dioxide and/or water, and sometimes ammonia, HCl and/or hydrocarbons other than methane. In one embodiment, the incoming gas comprising monocyclic aromatic compounds and tar-like components, in which it is treated according to the second embodiment wherein steps (a") and (b") are implemented. In one embodiment, the incoming gas comprising monocyclic aromatic compounds and no tar-like components, in which it is preferably treated according to the second preferred embodiment wherein steps (a") and (b") are not implemented.

In the context of the present invention, aromatic compounds include both compounds having only carbon atoms in the aromatic ring(s) (i.e. carbocycles) as well as heteroaromatic compounds comprising at least one non-carbon ring atom (i.e. heterocycles), typically selected from N, O and S. The monocyclic aromatic compounds comprised in the incoming gas which is subjected to step (a) are generally monocyclic and low boiling, while the content of high boiling aromatic compounds is preferably kept low. The monocyclic aromatic compounds present in the incoming gas typically have a boiling point below 200 °C (when determined at ambient pressure), preferably below 120 °C, most preferably below 100 °C. The monocyclic aromatic compounds include all of benzene, toluene, ethylbenzene and xylene, which are together referred to as BTEX. In addition, in case a detarred gas is subjected to step (a), e.g. in the context of the second embodiment, further low-boiling monocyclic aromatic compounds, such as styrene, cresol and thiophene, may be present in the detarred gas and end up in the fraction of aromatic compounds isolated in step (c). In case the gas, comprises aromatic compounds having a boiling point in the range of 100 - 300 °C, these compounds may be present in the gas subjected to step (a) or they may be removed in a pre-washing or pre-cleaning step prior to step (a). Examples of such aromatic compounds include phenol, cresols, styrene, cumene, naphthalene and the like.

Instead of or, most commonly, in addition to, the monocyclic aromatic compounds, tar-like components may be present. In case tar-like components are present, the gas is preferable subjected to detarring according to the first embodiment and optionally subsequently to BTEX scrubbing. In other words, in the context of the second embodiment, the gas being subjected to step (a) is conveniently lean in tar-like components. In one embodiment, the gas subjected to step (a) is substantially free from tar-like components. In case the incoming gas contains substantial amounts of tar-like components, they are beneficially removed from the incoming gas prior to step (a). In that case, the process according to the second embodiment comprises a step (a') of removing tar-like components from the gas (detarring). Tar-like components are aromatic compounds having a boiling point above 300 °C (when determined at ambient pressure), preferably above 250 °C, most preferably above 200 °C. Aromatic compounds that are classified as tar-like in the context of the present invention usually are polycyclic, such as bicyclic, tricyclic and tetracyclic aromatic compounds having 9 - 24 aromatic ring atoms, typically 9 - 18 carbon atoms. These are largely carbocyclic (i.e. all-carbon aromatic rings), but heterocyclic compounds such as indoles, (iso)quinolines and chromenes, may also be present. Examples of (polycyclic) aromatic compounds which are classified as tar-like components include indene, naphthalene, quinoline, isoquinoline, 2-methylnaphthalene, 1-methyl-naphthalene, biphenyl, ethenyl-naphthalene, acenaphthylene, acenaphthene, fluorene, anthracene, phenanthrene, fluoranthene, pyrene, aceanthrylene, benzoanthracenes and chrysene. In the context of the present invention, phenol is also considered a tar-like component, even though it is monocyclic, since it is largely removed during the detarring step. In addition to the 9 - 18 ring atom components, heavier aromatic compounds, e.g. up to 24 ring atoms or even larger are also classified as tar-like in the context of the present invention. Examples include benzofluoranthenes, benzopyrenes, perylene, picene, benzoperylenes, indenoperylenes, dibenzoanthracenes, benzoperylenes, coronene, etc. Preferably, their content in the gas subjected to step (a) is kept low and/or they are all effectively removed in a pre-cleaning step. Otherwise, they would be removed in detarring step (a) or (a"). In case the incoming gas stream contains substantial levels of tar-like components having more than 18 ring atoms, in particular if it contains components of more than 24 ring atoms, a pre-cleaning step is preferably incorporated in the present process, as further described below.

Lighter aromatic compounds such as phenol, cresols, styrene, cumene and the like may also be substantially absent in the gas subjected to BTEX scrubbing step (a) or (a'), e.g. by removal thereof in detarring step (a) or (a"), although they may also be present in the gas and removed in the BTEX scrubbing step. It depends on the exact conditions of the detarring step whether or not these lighter aromatic compounds are removed in the detarring step. If so, those are then included in the tar-like component fraction removed in the detarring step. However, they may also be present in the gas subjected to the BTEX scrubbing step, either when a detarring step is not performed or in case the conditions of the detarring step are such that these compounds are not removed. As such, these compounds will be removed in the BTEX scrubbing step and are thus included in the fraction of monocyclic aromatic compounds obtained in step (b) or separated in step (c).

The incoming gas typically comprises at least 30 vol.%, especially at least 40 vol.%, of one or more components selected from hydrogen, methane and carbon monoxide. A typical composition of a gas comprises 10 - 40 vol.% CO, 5 - 30 vol.% CO₂, 2 - 75 vol.% H₂, 4 - 24 vol.% CH₄ and 0 - 40 vol.% H₂O, based on total volume of the gas excluding inert gases such as nitrogen. In one embodiment, the gas comprises 10 - 40 vol.% CO, 5 - 30 vol.% CO₂, 2 - 75 vol.% H₂, 4 - 24 vol.% CH₄ and 5 - 40 vol.% H₂O, based on total volume of the gas excluding inert gases such as nitrogen. Inert gases may be present e.g. up to 70 vol.%, preferably 30 - 60 vol.%. Based on total volume, the gas preferably comprises 5 - 40 vol.% CO, 2 - 30 vol.% CO₂, 1 - 40 vol.% H₂, 2 - 24 vol.% CH₄, 0 - 40 vol.% H₂O and 0 - 60 vol.% inert gases, preferably N₂, or even 5 - 40 vol.% CO, 2 - 30 vol.% CO₂, 1 - 40 vol.% H₂, 2 - 24 vol.% CH₄, 2 - 40 vol.% H₂O and 0 - 60 vol.% inert gases, preferably N₂. Monocyclic aromatic compounds may be present in widely varying concentration, e.g. from 1 ppm to 5 vol.%, typically from 10 ppm to 2 vol%, of which benzene typically is the most abundant one (e.g. up to 3 vol.%). In case the incoming gas stream originates from gasification of wood, it typically contains 5000 - 10000 ppm benzene, while the benzene content may be as high as 3 vol.% in case the incoming gas stream originates from gasification of organic waste. Despite their often relative low concentration, tar-like components and monocyclic aromatic compounds represent a significant part of the energetic content of the gas. A content of about 1 vol% of monocyclic aromatic compounds represents about 10 % of the energetic content of the gas. Tar-like components may e.g. be present in the range of at most 2 vol%, preferably at most 0.5 vol.%, most preferably in the range of 1 ppm to 0.1 vol%. In terms of weight per volume, tar-like components are preferably present in the incoming gas that is subject to the BTEX scrubbing step in an amount of at most 100 mg per m³, preferably 1 - 80 mg per m³, most preferably 5 - 50 mg per m³, when determined at ambient temperature and pressure.

In case the process according to the invention comprises a BTEX scrubbing step, the preferred tar content of the gas which is subjected to the BTEX scrubbing step is conveniently described by the tar dew point of the gas. The tar dew point is known in the art to be the temperature at which the real total partial pressure of tar equals the saturation pressure of tar (see e.g. Rabou et al., Energy Fuels, 2009, 23, 6189-6198). Preferably, the tar dew point of the gas subjected to the BTEX scrubbing step is at most 60 °C, more preferably in the range of -10 °C to 50 °C. In a preferred embodiment, the tar dew point is not higher than the temperature at which the BTEX scrubbing step is performed. As such, condensation of tars during the contacting of the BTEX scrubbing step is prevented and the fraction of monocyclic aromatic compounds, which is removed from the gas in the BTEX scrubbing step and isolated in step (c), is not contaminated with high boiling tar-like components. Likewise, it is preferred that the water dew point of the gas subjected to the BTEX scrubbing step is not higher than the temperature at which the BTEX scrubbing step is performed. A higher water dew point leads to condensation of water during the contacting of step (a), which hampers absorption of the monocyclic aromatic compounds in the washing liquid. The washing liquid is not miscible with water, such that a two-layer system is formed, in which the contacting between the incoming gas and the washing liquid is reduced. In a preferred embodiment, the water content of the incoming gas that is subjected to the BTEX scrubbing step is at most 10 vol.%, more preferably 0.1 - 5 vol.%, most preferably 0.5 - 4 vol.%, based on total volume.

The incoming gas preferably originates from gasification of biomass, waste or a mixture thereof. The biomass may be agricultural or forestry residues (wood chips, leafs, straw, grass, etc.), organic waste (e.g. be municipal solid waste (MSW) and/or materials resulting in refuse-derived fuel (RDF)) and the like. In an especially preferred embodiment, the incoming gas stream is an energy gas originating from gasification of biomass. Such gasification is known in the art. The present process is particularly suited to clean energy gases originating from an indirect gasifier, as therein larger quantities of benzene are produced than during regular gasification. The skilled person finds further guidance on gasification and pyrolysis, especially indirect gasification, in WO 2008/108644 and WO 2007/061301.

Gasifiers are typically used in the production of heat, electricity, Fischer-Tropsch Diesel or substitute natural gas (SNG). Preferably, the process according to the invention is implemented in the production of electricity or SNG, most preferably SNG. Preferably, the gas originates from biomass. It is thus most preferred that the gas is used for producing bio-SNG. In the production of SNG, all components (other than syngas and methane) should be removed or converted to syngas (a mixture of H₂, CO and possibly some CO₂) and subsequently to methane. Moreover, when the gas is to be used as fuel, the presence of aromatic compounds, including monocyclic aromatic compounds such as benzene, is highly undesirable, as these are typically not (completely) combusted and end up in the flue gas. Conventional tar-scrubbing techniques, as described in the background, are not capable of removing sufficient amounts of monocyclic aromatic compounds such as benzene. Consequently, in the production of SNG, the (detarred) gas needs to be subjected to reforming using large amounts of steam. This is a high energy consuming process to produce syngas from monocyclic aromatic compounds such as benzene, and a further conversion of syngas to methane is also energy consuming. By removal of the monocyclic aromatic compounds using the process according to the invention, the overall yield of methane from biomass may be slightly reduced (as benzene is not converted to methane), but an additional valuable product in the form of benzene or BTEX is obtained and the overall energy efficiency of the process is greatly improved by eliminating two energy consuming process steps. This is particularly important for the conversion of biomass to bio-SNG. Moreover, the market value of biomass-based benzene or bio-based BTEX is greater than of biomass-based methane, which is an important factor in field of green energy. In order to be able to compete with and eventually replace conventional fossil fuel based energy, the generation of green energy should be as cheap and profitable as possible. Hence, it is highly desirable that the process according to the invention comprises a BTEX scrubbing step as defined herein, most preferably a BTEX scrubbing step according to the invention as defined by steps (a), (b) and (c).

### Possible pre-treatment of the gas

Depending on the exact composition of the gas to be subjected to the process according to the invention, pre-treatment of the gas may be preferred. The incoming gas, especially when originating from a gasification or pyrolysis reactor, is advantageously pre-treated prior to being subjected to step (a). Further pre-treatment, which may be applied upstream of step (a), can be used to remove non-gaseous components (e.g. particulate material) from the gas stream, high molecular weight tar-like components and/or polar molecules. Preferably, at least tar-like components of more than 24 ring atoms and particulate material are removed, when present, as such components may be detrimental for effective clearing of the gas using the process according to the invention.

A pre-cleaning step may be performed, which is especially preferred in case the incoming gas stream contains substantial levels of tar-like components having more than 18 ring atoms, in particular if it contains components of more than 24 ring atoms. Such a pre-cleaning step typically involves contacting the gas stream with another washing or scrubbing liquid prior to being contacted to the washing liquid in step (a). Herein, tar-like components are condensed and collected in the washing liquid, after which they may be separated from the spent washing liquid. The washing liquid to be used in the pre-cleaning step preferably has a high affinity for more complex polycyclic (aromatic) hydrocarbons. Suitable scrubbing liquids are aromatic hydrocarbons, for example polycyclic aromatic compounds corresponding to the heavy tar-like compounds present in the gas and/or originate from scrubbing the tar containing gas. They will be supplemented during scrubbing with tars extracted from the gas. The pre-cleaning can be performed at a higher temperature than step (a), for example between 150 and 900 °C, more typically using a liquid inlet temperature of between 150 and 300 °C, and a gas inlet temperature between 250 °C and 900 °C. Thus, in a preferred embodiment, the gas, prior to step (a) or (a"), is treated with a scrubbing liquid comprising aromatic hydrocarbons, at a temperature of between 150 and 900 °C.

In the pre-cleaning step using (poly)aromatic hydrocarbons as washing liquid, the spent washing liquid resulting from the contacting step can be subjected to a separation step using evaporation of the lighter components. These lighter components can be reused as absorption (scrubbing) liquid for the pre-cleaning step. The heavier fraction resulting from the separation step may be discharged. Part of this discharged heavy fraction can be returned to the inlet of a gasification reactor, in case the incoming gas stream originates from such a reactor, and subjected (again) to gasification to be converted into lighter components, or it can be used for other purposes such as heating. Further details of the pre-cleaning step using aromatic hydrocarbons are described in WO 2011/037463.

Alternatively or additionally, the gas may be cleared from dust particles and/or other particulate material before being subjected to step (a) and optionally step (a"), possibly before and/or after the optional pre-cleaning step. Clearing from dust particles may be performed by passing the gas e.g. through an electrostatic filter or an electrostatic precipitator as described in WO 2008/010717. Other methods or devices for clearing dust particles can be used as well, such as an aerosol scavenger as described in WO 2011/099850.

If desired, a further cleaning step can be introduced before or after step (a), for example for (further) removing polar compounds such as ammonia and the like, using neutral, acidic or alkaline aqueous scrubbing liquids, or for removing water, e.g. by condensation. Such condensation is advantageously implemented between a detarring step and a BTEX scrubbing step. However, appreciable levels of water, HCl, ammonia, amines and the like are effectively captured by the (pre-)washing liquid, so that additional cleaning steps will often not be necessary. Compounds such as water and ammonia are readily desorbed from the scrubbing liquid, leaving the scrubbing liquid clean and stable. Thus, in one embodiment, the process according to the invention does not comprise a step wherein the gas is contacted with neutral, acidic or alkaline aqueous scrubbing liquids. In one embodiment, water and other polar components such as HCl, ammonia are condensed from the gas. Such a condensation step is preferably performed prior to a BTEX scrubbing step, most preferably between a detarring step and a BTEX scrubbing step. Any water present in the detarring gas is thus removed, which was found to improve the performance during the BTEX absorbing step.

### The washing liquid

The washing liquid that is used in the process according to the invention typically comprises or is an organic solvent, such as aliphatic or aromatic solvents, preferably aliphatic or aromatic hydrocarbons. Typically, an oil is used that is liquid at the conditions at which step (a) is performed. Such washing liquids are known in the art and any solvent which is known in the art to be suitable in this respect, such as mineral, vegetable or synthetic oils, may be used in the washing liquid. Preferably, the washing liquid comprises at least 75 vol.% of the solvent, more preferably at least 90 vol.% or even at least 95 vol.% of the solvent. It is most preferred that the washing liquid is the solvent as specified herein. In a preferred embodiment, both the washing liquid and the pre-washing liquid contain the same solvent. It is preferable to use a non-volatile oil, and more particularly an oil in which the molecules comprise approximately 15 - 50 carbon atoms. The term non-volatile is understood as meaning an oil grade which, at a temperature of 70 °C, preferably at a temperature of 100 °C, releases less than 10 mg of oil per standard cubic metre of cleaned gas. Preferred solvents are selected from hydrocarbon oils (e.g. petroleum oil, coal tar fractions), biomass-derived oils (e.g. vegetable oil, biodiesel, waste cooking oil, wood pyrolysis oil) and silicon oils. In one embodiment, the solvent is a polysiloxane or a mineral aliphatic oil, most preferably a polysiloxane is used. Polysiloxanes have been found especially suitable to be used as washing liquid in step (a), especially in view of the elevated temperatures used herein and in the subsequent stripping step (b). Mineral and hydrocarbon oils are typically more volatile than the polysiloxane, which hampers stripping especially when steam is used as stripping gas, as steam has a much higher boiling point compared to other suitable stripping gases such as air, nitrogen and/or carbon dioxide.

Thus, in an especially preferred embodiment, the washing liquid comprises or even consists of organic polysiloxanes, preferably a polymethylphenylsiloxane or a polydiphenyldimethylsiloxane, most preferably a polymethylphenylsiloxane. The organic polysiloxane typically comprises an alternating silicon-oxygen chain wherein the silicon is further substituted with organic groups. The organic polysiloxane may also be referred to as polydiorganyl-siloxane. Preferably the polysiloxane is an aryl polysiloxane, i.e. at least a part of the organic groups are aryl groups, including aralkyl or alkaryl groups. Preferably, the polysiloxane comprises an average of between 0.2 and 1.8 C₅-C₁₄ aryl groups, more preferably 0.2 - 1.8 C₅-C₁₀ aryl groups, per silicon atom. Advantageously, the polysiloxane also comprises alkyl groups, in particular 0.2 - 1.8 C₁-C₆ alkyl groups per silicon atom, more in particular 0.2 - 1.8 C₁-C₄ alkyl groups per silicon atom. In a preferred embodiment, the polysiloxane comprises an average of between 0.5 and 1.5 C₅-C₁₄, more preferably 0.5 - 1.5 C₅-C₁₀ aryl groups, most preferably 0.5 - 1.5 C₆-C₈ aryl groups per silicon atom and/or between 0.5 and 1.5 C₁-C₄ alkyl groups per silicon atom. The polysiloxane preferentially has a molar weight between 500 and 14,000 g/mol, more preferably between 700 and 7,000 g/mol, most preferably between 1,000 and 5,500 g/mol. The polysiloxane preferably has a narrow size distribution (dispersity), such as a dispersity *M_{w}* / *M_{w}* in the range of 1 - 5, preferably 1 - 2, or even 1 - 1.2. Such low dispersity enables the use of stripping as efficient clearing of the spent washing liquid. Washing liquids with a broad MW distribution cannot be efficiently stripped and typically require distillation for the contacting of step (b). Furthermore, relatively small molecules of washing liquid would evaporate during stripping and end up in the loaded stripping gas, hampering the separation of monocyclic aromatic compounds from the stripping gas in step (c) and consequently polluting the composition of monocyclic aromatic compounds and/or the stripping gas that may be reused in step (b). The presence of relatively large molecules of washing liquid impart a high viscosity on the washing liquid, which hamper the process and requires greater temperatures for efficient stripping, as such reducing the efficiency of the process. Hence, a narrow size distribution is preferred fpr the polysiloxane washing liquid. Within the MW ranges defined above, the process according to the invention is performed most optimally.

The polysiloxane can be represented by one of the formulas (I) and (II):

-[SiR¹R²-O]ₙ- (I)

-[Si(R¹)₂-O]ₙ₁[Si(R²)₂-O]ₙ₂[SiR¹R²-O]ₙ₃- (II).

Herein,
- each occurrence of R¹ and R² is individually selected from optionally substituted C₁-C₁₄ hydrocarbyl groups,
- n is an integer in the range of 5 - 100, preferably in the range of 7 - 40,
- n1 + n2 + n3 equals n, wherein
   - (n1 + n3)/n2 is in the range of 1/9 - 9/1, preferably in the range of 1/4 - 4/1, most preferably in the range of 2/3 - 3/2, and/or
   - (n2 + n3)/n1 is in the range of 1/9 - 9/1, preferably in the range of 1/3 - 3/1, most preferably in the range of 2/3 - 3/2.

Although any hydrocarbyl group known in the art may be suitable, "hydrocarbyl" typically refers to alkyl or aryl. In the context of the present invention, the term "alkyl" includes linear, branched and cyclic alkyl moieties. In the context of the present invention, the term "aryl" includes alkaryl moieties, wherein an alkyl moiety is substituted with an aryl moiety, and aralkyl moieties, wherein an aryl moiety is substituted with an alkyl moiety. The hydrocarbyl moieties may be unsubstituted or substituted, preferably they are unsubstituted. The optional substituents that may be present on the hydrocarbyl groups are preferably selected from hydroxy, alkoxy (e.g. C₁-C₄ alkoxy), halogen, alkanoyl or acyl (e.g. C₁-C₄ alkanoyl), carboxy, alkanoyloxy (e.g. C₁-C₄ alkanoyloxy), alkoxycarbonyl (e.g. C₁-C₄ alkoxycarbonyl), amino (primary, secondary or tertiary), carbamoyl (e.g. C₁-C₄ carbamoyl), cyano, nitro. Preferably, the hydrocarbyl is unsubstituted or contains an alkoxy substituent, preferably methoxy.

Preferred polysiloxanes of formulas (I) and (II) are those wherein at least 50 % (by number) of the occurrences of R¹ are aryl groups, preferably aryl groups having 5 - 14 carbon atoms, more preferably 5 - 10 carbon atoms, most preferably of 6 - 8 carbon atoms, the remaining occurrences of R¹ preferably being alkyl groups. Preferably, at least 80 % of the occurrences of R¹, more preferably at least 95 % of of the occurrences of R¹ are such aryl groups. Aryl groups as used herein include alkylaryl (alkaryl) and arylakyl (aralkyl) groups. Especially preferred aryl groups include phenyl, methylphenyl (toly1, preferably p-tolyl), methoxyphenyl, benzyl, 2-phenyl-isopropyl, naphthyl and the like. Preferred polysiloxanes of formulas (I) and (II) comprise C₁-C₄ alkyl groups as R², most preferably the alkyl groups include methyl and ethyl.

The polysiloxane may be a homopolymer or an alternating, block or random copolymer of dialkyl, alkylaryl and/or diaryl siloxanes. The polysiloxane homopolymer is represented by formula (I), wherein each occurrence of R¹ is the same and each occurrence of R² is the same. The polysiloxane copolymer is represented by formula (II). The polysiloxane can also be a mixture of different poly-dialkyl, polyalkyl-aryl and/or polydiaryl siloxanes. Preferably, the polysiloxane is a poly-methyl-phenyl-siloxane (i.e. formula (I), wherein R¹ = Ph and R² = Me) or a poly-diphenyl-co-dimethyl-siloxane (i.e. formula (II), wherein R¹ = Ph, R² = Me and n3 = 0) or a poly-(methyl-phenyl)-co-dimethyl-siloxane (i.e. formula (II), wherein R¹ = Ph,R² = Me and n1 = 0). Preferably the proportion of aryl groups is between 0.5 and 1.5 aryl group per silicon atom. A most preferred polysiloxane is poly(methylphenylsiloxane). Depending on the tar components to be removed and the removal conditions, specific siloxanes, e.g. having specific molecular weight and/or specific organic groups can be prepared and used. Polysiloxanes can be prepared by methods known in the art. Various organic polysiloxanes including poly(methylphenylsiloxane) and poly-diphenyl-co-dimethyl-siloxane are commercially available.

The washing liquid is used in both step (a) and preferably in step (a) or (a") for contacting with the gas stream. Although the washing liquid used in the two steps may be different, it is preferred that the same polysiloxane is used in both steps. Preferably, the polysiloxan as defined herein is used in both steps, most preferably poly(methylphenylsiloxane) is used in both steps.

### Contacting step (a)

In step (a) of the process according to the invention, the gas is contacted with the washing liquid at a temperature in the range of 15 - 60 °C, to absorb therein the aromatic compounds. According to the first embodiment outside of the scope of the invention, step (a) is performed at a temperature of 60 - 250 °C, preferably 90 - 220 °C, more preferably 120 - 190 °C, and the absorbed aromatic compounds include tar-like components, such as polycyclic aromatic compounds and phenol. According to the second embodiment, which is according to the invention, step (a) is performed at a temperature of 15 - 60 °C, preferably 25 - 50 °C, more preferably 30 - 40 °C, and the absorbed aromatic compounds include monocyclic aromatic compounds such as benzene, toluene, ethylbenzene and xylene. In one embodiment, step (a) is performed at a temperature in the range of 31 - 50 °C, preferably 32 - 48 °C, most preferably 33 - 45 °C.

In step (a), aromatic compounds are removed from (washed from or scrubbed from) the gas. The contacting of step (a) affords a purified gas, which is depleted in aromatic compounds, and a spent washing liquid, which comprises the aromatic compounds. The spent washing liquid comprising aromatic compounds is stripped in step (b). The purified gas is one of the products of the process according to the invention. The purified gas discharged from a BTEX scrubbing step typically contains the following components: H₂, CO, CH₄, often some small hydrocarbons containing two or three carbon atoms, optionally small sulphur species such as H₂S, COS, CS₂ and possibly minor amounts of small organic sulphur compounds (e.g. methyl mercaptan), and optionally traces of HCl and ammonia. The purified gas discharged from a detarring step, also referred to as a detarred gas, further typically contains monocyclic aromatic compounds, which are preferably removed in a BTEX scrubbing step. The purified gas may optionally be further cleaned or converted as explained below.

The process may also comprise a step (a') or (a"), as defined further above. This step is preferably performed in the same way as step (a). Thus, everything described for step (a) herein and preferred embodiments thereof preferably equally applies to step (a') as well as step (a").

### Stripping step (b)

After being contacted with the gas, the spent washing liquid containing the aromatic compounds is stripped, i.e. the aromatic compounds are separated (or removed) from the spent washing liquid, by contacting with a stripping gas. The contacting of step (b) may also be referred to as "stripping". Stripping is known in the art. As such, the stripped washing liquid may advantageously be used again as washing liquid in step (a), or even as pre-washing liquid or post-washing liquid in case respectively step (a") or step (a') is performed with the same washing liquid. Thus, in a preferred embodiment, the spent washing liquid comprising the monocyclic aromatic compounds is stripped and subsequently returned for a further contact cycle of step (a) or to the contacting of step (a') or (a"), preferably to step (a). Stripping step (b) further affords a loaded stripping gas, which comprised the aromatic compounds.

Stripping step (b) preferably involves heating the spent washing liquid and/or lowering the pressure. In one embodiment, stripping is performed at a temperature in the range of 80 to 250 °C, preferably 120 to 220 °C. Most optimal stripping was observed at a temperature of 160 - 180 °C. Preferably, the temperature is raised to at least 50 °C, preferably in the range of 80 °C to 120 °C, above the contacting temperature of step (a). A temperature increase of 20 °C more or less corresponds to a pressure decrease of a factor 2 and a temperature increase of at least 50°C corresponds to a pressure decrease of a factor of at least 5.6. Thus, at equal temperatures, the stripping is preferably performed at a pressure which is a factor of at least 5.6, more preferably a factor between 16 and 80, below the scrubbing pressure. Also, a temperature increase may be accompanied with a pressure decrease, wherein both the temperature increase and the pressure decrease may be performed to a lower extent. In absolute terms, the stripping can be performed for example at a temperature between 75 and 250 °C, in particular between 100 and 220 °C, most preferably in the range of 140 - 180 °C at atmospheric pressure. The inventors surprisingly found that at a temperature above 140 °C and atmospheric pressure stripping of the spent washing liquid could efficiently performed with a stripping gas flow as low as 0.25 m³/h. Such low flow rates are preferred, as the amount of steam needed is reduced and the separation of step (c) is facilitated. In a preferred embodiment, the temperature is more or less equal within the stripping unit, e.g. in the stripping column or tower. In other words, preferably no gradient is present. The inventors found that a constant temperature throughout the stripping unit ensures that the greatest amount of monocyclic aromatic compounds are separated from the spent washing liquid. As such, the stripped washing liquid can readily be reused in step (a) or optionally step (a') or (a"). The temperature difference within the stripping unit is preferably below 25 °C, more preferably below 10 °C, or even below 5 °C. The absence of a temperature gradient may be accomplished by hearing the spent washing liquid and the stripping gas to about the same temperature before they are introduced in the tripping unit.

During stripping, the spent washing liquid is contacted with a stripping gas. The stripping gas comprises at least 50 vol.% steam, and may contain further gaseous components such as air, carbon dioxide, nitrogen or mixtures thereof. Preferably, the stripping gas comprises at least 80 vol.% steam, more preferably at least 90 vol.% steam or even at least 95 vol.% steam. It is most preferred that the stripping gas is steam, i.e. contains about 100 vol.% steam. Pure steam is especially preferred, as it facilitates the separation of the stripping gas, the aromatic compounds and possibly any remaining permanent gases in step (c). Thus, the loaded stripping gas is subjected to separation step (c).

The skilled person is capable of adjusting the flow rate of the stripping gas in step (b) to obtain efficient stripping. The efficacy of the stripping may for example depend on the temperature at which step (b) is performed. For example, a reduced temperature is preferably accompanied with an increased flow rate, and vice versa. In a continuous process, the flow rate is conveniently set such that the amount of stripped washing liquid per unit of time more or less equals the required amount of the washing liquid to efficiently scrub the incoming gas. The flow rate wherein step (b) is performed is preferably 0.1 - 5 times greater, more preferably 0.5 - 2 times greater, than the flow rate at which step (a) is performed. Using such flow rates, a sufficient amount of washing liquid is stripped in step (b) for efficient washing of the gas in step (a), such that step (a) may be performed continuously. Efficient continuous stripping was observed using flow rates of 0.25, 0.5 and 1 m³/h steam. Preferably, a flow rate or 0.02 - 5 m³/h steam, more preferably 0.1 - 0.5 m³/h steam, most preferably 0.2 - 0.4 m³/h steam is used during step (b). Low flow rates are preferred, as the amount of steam needed is reduced and the separation of step (c) is facilitated.

The process may also comprise a step (b') or (b"), as defined further above. Step (b') or (b") may be performed with a stripping gas comprising less than 50 vol.% steam or even no steam at all, but apart from the stripping gas, all said above for step (b) preferably equally applies to step (b') as well as step (b"). Any stripping gas known in the art may be used in step (b') or (b"), and suitable options include air, carbon dioxide, nitrogen, steam or mixtures thereof. Preferably, the stripping gas used in step (b') or (b") is steam, nitrogen or air. Most preferably, the stripping gas used in step (b') comprises steam, as defined above for step (b). In one embodiment, the stripping gas used in step (b") comprises steam, as defined above for step (b). The use of steam in step (b') or (b") provides the same advantages as described herein for the use of steam in step (b). In one embodiment, the stripping gas used in step (b") comprises nitrogen or air. The use of nitrogen or air as stripping gas in step (b") is especially preferred in case the process according to the invention is implemented in a gasification or pyrolysis process. The loaded pre-stripping gas, which comprises the tar-like components, is advantageously recycled to the gasification or pyrolysis reactor, either as feedstock to convert the tar-like components into valuable compounds for e.g. SNG or as fuel for heating the reactor. In such an environment, the presence of steam is undesirable. Alternatively, the tar-like components are separated from the pre-stripping gas, in step (c"). In case the tar-like components are separated from the loaded pre-stripping gas, a cleared pre-stripping gas and a fraction comprising tar-like components is obtained. The cleared pre-stripping gas is preferably reused in another cycle of step (b").

### Separation step (c)

The loaded stripping gas is subjected to separation step (c). Herein, the aromatic compounds are separated from the stripping gas. Separation step (c) affords the stripping gas and an oily fraction of aromatic compounds. The stripping gas is preferably reused in another cycle of step (b). Step (c) is effectively performed by condensation of the steam and the aromatic compounds comprised in the loaded stripping gas, providing an immiscible composition. The immiscible composition is a composition comprising two phases, either a liquid phase and a gaseous phase or two immiscible liquid phases (an aqueous phase and an oily phase). Herein, immiscible refers to the inability of the two phase to form a homogenous mixture at the relevant conditions at which step (c) is performed. Both the steam and the aromatic compounds are condensed. Separation is readily accomplished with oil/water separation. Condensation of the loaded stripping gas comprising water and aromatic compounds and collection thereof in a vessel allows for liquid-liquid separation thereof, as known in the art. Such separation is conveniently based on refractive index. In the context of the present invention, the term "stripping gas" is used for the gas used to strip the washing liquid, also after condensation thereof such as in step (c). Stripping gas may thus refer to a liquid such as water.

Condensation may be accomplished by any means known in the art, such as lowering the temperature and/or increasing the pressure. Condensation is preferably accomplished by lowering the temperature of the stripping gas comprising the aromatic compounds to below the dew point of water. Conveniently, the temperature is reduced to 3 - 80 °C, preferably 4 - 50 °C. For a detarring step (a) or (a"), the temperature is preferably reduced to 10 - 80 °C, preferably 15 - 50 °C. For a BTEX scrubbing step (a) or (a'), the temperature is preferably reduced to 4 - 40 °C, more preferably to 5 - 30 °C, preferably at ambient pressure. Ambient temperature (typically 15 - 25 °C) may conveniently be used, as no active cooling (to below ambient temperature) is required. However, using further reduced temperatures such as 4 - 15 °C, preferably 5 - 8 °C may be also convenient, as the amount of gaseous water and aromatic compounds is further reduced. At such reduced temperature, both the water and the aromatic compounds are typically predominantly in the liquid phase (although some remaining gaseous molecules may be present), but in view of their immiscible character, they form a two-layer system which is readily separated based on refractive index. Especially in case of a BTEX scrubbing step, cooling to below 4 °C is not advantageous, as the mixture of monocyclic aromatic compounds, typically containing benzene as major component, solidifies below 4 °C, which hampers separation. The melting temperature of the mixture of monocyclic aromatic compounds is typically slightly below that of benzene (*T*ₘ = 5.5 °C) as the presence of other (monocyclic aromatic) compounds reduces the melting temperature compared to pure benzene. Typically, both the aqueous fraction and the organic fraction containing the aromatic compounds are drained separately.

According to a preferred embodiment of step (c), condensation is performed by two consecutive steps. First, the loaded stripping gas is subjected to condensation at a temperature of 15 - 40 °C, preferably 25 - 35 °C, more preferably at ambient temperature. Employing such temperatures reduces the operating costs associated with cooling the bulk of loaded stripping gas that is fed to step (c), while at the same time enables condensation of the majority of the stripping gas and the aromatic compounds comprised therein. The condensed liquids are subjected to liquid-liquid separation as described above, while the remainder of the loaded stripping gas, i.e. which is not condensed, is subjected to a second condensation step at a temperature of 4 - 10 °C, preferably 4 - 5 °C. At such temperatures, the remaining components of the loaded stripping gas are readily condensed, except for the permanent gases dissolved in the loaded stripping gas. As such, the operating costs for cooling are kept as low as possible, as only a minor part of the loaded stripping gas is subjected to the second condensation step, while efficient collection of substantially all of the aromatic compounds that are stripped from the washing liquid is readily accomplished. Herein, two immiscible liquid phases are obtained, which may be combined before or after separation into condensed stripping gas and the fraction of aromatic compounds. Preferably, the immiscible compositions are combined prior to separation thereof, such that only a single separation step is needed. Most conveniently, the immiscible composition obtained in the second condenser, which is typically colder and of less volume, is led to the immiscible composition of the first condenser, where separation takes place. An alternative approach is to combine both aqueous layers to form a single aqueous layer, which after draining and vaporization may be reused as stripping gas, while both organic layers are drained separately and optionally combined afterwards.

The drained aqueous fraction(s) consist(s) essentially of water and may be reused as stripping gas, after heating to above the dew point of water. The drained organic fraction(s) consist(s) essentially of the aromatic compounds. The fraction of aromatic compounds may comprise compounds like naphthalene, phenol and monocyclic aromatic compounds such as benzene, toluene, ethylbenzene and xylene. In case of a detarring step, the fraction typically contains tar-like components such as naphthalene and phenol. In case of a BTEX scrubbing step, the fraction contains monocyclic aromatic compounds and typically comprises at least 75 wt.% or at least 90 wt.% or even at least 95 wt.% of the combined content of benzene, toluene, ethylbenzene and xylene (BTEX), based on total weight of the organic fraction. Typically, the majority of the BTEX fraction is benzene, such as at least 60 wt.% or even at least 90 wt.%. The fraction of monocyclic aromatic compounds typically contains benzene, toluene, ethylbenzene, xylene, and possibly traces of other aromatic compounds such as styrene, cresol, naphthalene and thiophene. Herein, "traces" means less than 1 wt.%, based on total weight of the organic fraction. This organic fraction or "BTEX fraction" is a key product of the process of the invention.

In addition to a liquid aqueous phase (the stripping gas) and a liquid organic phase, a small percentage, typically less than 2 vol.%, of the loaded stripping gas that is obtained in step (b) is formed by permanent gases (i.e. compounds that remain gaseous even after reduction of the temperature below the dew point of water). During contacting step (a), minor amounts of these permanent gases (e.g. CH₄, CO, CO₂) may be absorbed by the washing liquid and led to step (b) where they are stripped together with the aromatic compounds. Typically, the permanent gas mainly contains CO₂, as that has the highest affinity for the washing liquid washing liquid. These gaseous compounds are preferably recombined with the gas before this is subjected to step (a). As such, any further remaining aromatic compounds in the gas phase are removed from this remaining gas stream in a further contacting step (a). As such, the maximal amount of valuable components are extracted from the incoming gas stream and the yield of the purified energy gas that is obtainable by the process according to the invention is maximized. At the same time contamination of the stripping gas is prevented, which may thus be used longer (i.e. more cycles) before the need of cleaning or discarding and replacing thereof.

The major advantage of using steam as stripping gas is that separation in step (c) is easily and efficiently accomplished, e.g. as described above by condensation of the stripping gas. In case a different stripping gas, such as air, nitrogen or carbon dioxide, would be used, this stripping gas would not be condensed in step (c) and thus classifies as a permanent gas. Separation between the stripping gas and the remaining gaseous compounds originating from the incoming gas stream (i.e. permanent gases) would not be feasible and valuable compounds would be lost in the cleared stripping gas. A further advantage of the use of steam as stripping gas in step (b) and the separation of step (c) is that any washing liquid, typically the polysiloxane, that may be led along with the loaded stripping gas from step (b) to step (c) is not lost (e.g. in a permanent gas stream or irreversibly deposited somewhere in the system), but may be recovered in separation step (c). The inventors found that traces of liquid polysiloxane accumulate as a third layer on the bottom of the collection vessel. Emptying this vessel for collection of the aromatic compounds and the condensed stripping gas allows for easy collection of these polysiloxane traces, which are thus not lost but may instead be reused as washing liquid.

The process may also comprise a step (c') or (c"), as defined further above. Although the exact manner on how the separation of step (c') or (c") is performed is not crucial for the operation of the process according to the invention, it is preferred that step (c') or (c") is performed in analogy with step (c), especially when step (b') or (b") is performed with a stripping gas comprising steam. Thus, all said above for step (c) preferably equally applies to step (c') as well as step (c").

### Post treatment

The purified gas, as obtained in step (a) or (a') of the process according to the invention, may be further treated, for example by sulphur removal and/or reforming. These two steps are commonly applied in the process of preparing (bio-)SNG to an energy gas obtained from gasification or pyrolysis. Herein, sulphur removal is typically accomplished by hydrodesulphurisation (HDS). During HDS, sulphur containing compounds such as thiols, thioethers and thiophenes are converted to H₂S, which is subsequently removed from the gas stream. Methods and means for HDS are known in the art and include catalytic or biological hydrogenation and subsequent removal of H₂S, e.g. by alkaline absorption or limited biological oxidation to produce elemental sulphur. However, in view of the removal of aromatic compounds according to the process according to the invention, both sulphur removal and reforming may not be required at all or needed to a lesser extent, compared to prior art processes. As such, sulphur removal may be accomplished efficiently by adsorption, e.g. to activated carbon, which is significantly more efficient in terms of costs and energy than HDS.

Step (a), especially when performed as BTEX scrubbing step, already removes the majority of the thiophene from the incoming gas stream, such that sulphur removal may not be needed, or only for the removal of sulphur-containing organic compounds smaller than thiophene (e.g. methyl or ethyl mercaptan, dimethyl sulphide), if present at all. HDS may be performed as sulphur removal step, for the removal of sulphur containing compounds such as methyl or ethyl mercaptan and dimethyl sulphide. As removal of thiophene is not needed, and thiophene is among the most challenging compounds to be removed by HDS, the HDS post treatment of the purified gas may be more flexible in terms of temperatures employed and type of catalyst used, compared to prior art processes. Depending on the composition of the incoming gas stream, sulphur capture may alternatively be performed by contacting the purified gas with activated carbon or may not even be needed altogether.

The purified gas may be subjected to (steam) reforming, wherein the remaining hydrocarbon compounds in the purified gas, typically containing 2 to 4 carbon atoms, are converted into H₂ and CO and/or CO₂ (syngas), and subsequently into methane (methanation), to obtain (bio-)SNG. Steam reforming is performed in the presence of steam and a metal-based catalyst, often nickel-based. Reforming is typically performed after sulphur removal, if sulphur removal is performed at all, as sulphur will poison the reforming catalyst. Since tar-like components, including light tars such as benzene, are effectively removed from the gas stream prior to reforming, reforming of the purified gas is much easier compared to reforming in prior art processes for the production of (bio-)SNG. Therein, compounds like benzene need to be converted into syngas, which places a greater burden on the reforming step in terms of energy and catalyst needed. Removal of benzene is also needed when the gas originating from biomass and/or coal gasification or pyrolysis is used as fuel in e.g. a gas engine, as such engines typically do not combust benzene (completely) which would lead to too much emission of benzene into the environment. Conventionally, benzene is removed by steam reforming, which may be replaced by the process according to the invention, which effectively removes benzene from gas streams.

As alternative to reforming, hydrocarbon compounds containing two or more carbon atoms may be separated from the purified gas, after which mainly H₂, CO, CO₂ and CH₄ remain. The remaining purified gas stream may be subjected to methanation to obtain (bio-)SNG. Such separation of hydrocarbons is known in the art and typically involves the use of a deethaniser, depropaniser, debutaniser, C₂- and C₃-splitters, and the like.

### System

Outside of the invention is an apparatus or system specifically designed to implement the process according to the invention. The system is a modular system, in which at least three, preferably at least four, five or even six modules (or units) are in fluid connection with each other. Herein, each module may be a separate unit or two or more modules may be integrated as a single unit. Preferably, each module is a separate unit and is distinguishable as such in the system. The modular system for performing the process according to the invention is suitably incorporated into a (biomass) gasification plant or a pyrolysis plant. The system is described with reference to the accompanying figure.

The modular system comprises:
(a) an absorbing unit comprising a gas inlet (a1) for receiving an gas comprising aromatic compounds, a liquid inlet (a2) for receiving a washing liquid, a gas outlet (a4) for discharging a purified gas and a liquid outlet (a5) for discharging a spent washing liquid;
(b) a stripping unit, comprising a liquid inlet (b1) for receiving the spent washing liquid, a gas inlet (b2) for receiving a stripping gas comprising steam, a gas outlet (b3) for discharging a loaded stripping gas and a liquid outlet (b4) for discharging a stripped washing liquid; and
(c) a separating module, comprising a gas inlet (c1) for receiving a loaded stripping gas, means (c2) for condensing steam and/or aromatic compounds from the loaded stripping gas, an outlet (c3) for discharging a cleared stripping gas, and an outlet (c4) for discharging the aromatic compounds, wherein at least one of outlets (c3) and (c4) is a liquid outlet.

In the system the different modules are interconnected, i.e. the outlet of one module is in fluid connection with the inlet of another module, preferably by means of a conduit. As such, constant flow of (liquid and gas) streams through the system is enabled. Thus, outlet (a5) is in fluid connection with inlet (b1) and preferably outlet (b4) is in fluid connection with inlet (a2). Likewise, outlet (b3) is in fluid connection with inlet (c1) and outlet (c3) is preferably in fluid connection with inlet (b2).

In accordance with the first embodiment of the process in a first embodiment of the system further comprises:
(a') a post-absorbing unit comprising a gas inlet (a'1) for receiving the purified gas, a liquid inlet (a'2) for receiving a post-washing liquid, a gas outlet (a'4) for discharging a purified gas and a liquid outlet (a'5) for discharging a spent post-washing liquid;
(b') a post-stripping unit, comprising a liquid inlet (b'1) for receiving the spent post-washing liquid, a gas inlet (b'2) for receiving a post-stripping gas, a gas outlet (b'3) for discharging a loaded post-stripping gas and a liquid outlet (b'4) for discharging a stripped post-washing liquid; and preferably
(c') a separating module, comprising a gas inlet (c'1) for receiving a loaded post-stripping gas, means (c'2) for separating the post-stripping gas from the monocyclic aromatic compounds, an outlet (c'3) for discharging a cleared post-stripping gas, and an outlet (c'4) for discharging the monocyclic aromatic compounds.
Herein, outlet (a4) is in fluid connection with inlet (a'1), outlet (a'5) is in fluid connection with inlet (b'1), outlet (b'3) is preferably in fluid connection with inlet (c'1) outlet (b'4) is preferably in fluid connection with inlet (a'2) and outlet (c'3) is preferably in fluid connection with inlet (b'2).

In accordance with the second embodiment of the process in a second embodiment of the system the system further comprises:
(a") a pre-absorbing unit comprising a gas inlet (a"1) for receiving an gas comprising tar-like components, a liquid inlet (a"2) for receiving a pre-washing liquid, a gas outlet (a"4) for discharging a pre-purified gas comprising monocyclic aromatic compounds and a liquid outlet (a"5) for discharging a spent pre-washing liquid;
(b") a pre-stripping unit, comprising a liquid inlet (b"1) for receiving the spent pre-washing liquid, a gas inlet (b"2) for receiving a pre-stripping gas, a gas outlet (b"3) for discharging a loaded pre-stripping gas and a liquid outlet (b"4) for discharging a stripped pre-washing liquid; and preferably
(c") a separating module, comprising a gas inlet (c"1) for receiving a loaded pre-stripping gas, means (c"2) for separating the pre-stripping gas from the tar-like components, an outlet (c"3) for discharging a cleared pre-stripping gas, and an outlet (c"4) for discharging the monocyclic aromatic compounds.
Herein, outlet (a"4) is in fluid connection with inlet (a1), outlet (a"5) is in fluid connection with inlet (b"1), outlet (b"3) is preferably in fluid connection with inlet (c"1) outlet (b"4) is preferably in fluid connection with inlet (a"2) and outlet (c"3) is preferably in fluid connection with inlet (b"2).

The system comprises at least modules (a), (b) and (c), most preferably the system comprises modules (a), (b), (c), as well as (a') and (b'), and preferably (c'), or (a") and (b"), and preferably (c").

### Absorbing unit (a)

The system comprises an absorbing unit (a) for absorbing the monocyclic aromatic compounds from the gas comprising aromatic compounds. Unit (a) comprises a gas inlet (a1) in one part, preferably the bottom part, of the unit for receiving the gas to be purified from monocyclic aromatic compounds, and a liquid inlet (a2) in another part, preferably the top part, of the unit for receiving the polysiloxane washing liquid. Thus, inlets (a1) and (a2) are preferably located in opposite parts of the unit (a). In case unit (a) is a unit for BTEX scrubbing, preferably a pre-absorbing unit (a') is present and inlet (a1) is in fluid connection with outlet (a'4). The connectivity between outlet (a'4) and inlet (a1) may contain a condenser (a8), a pump (a6), a safety filter (a7) and means (a3) for heating or cooling, typically a heat exchanger. Contact between the gas flowing in one direction, typically up-flowing gas, and the liquid flowing in another direction, preferably opposite direction (i.e. counter-current), typically down-flowing liquid, can be enhanced by conventional means such as by spraying, using a packed column or a plate column. Although co-current washing or scrubbing is feasible, a counter-current mode, preferably using a packed column, is preferred. Purified gas can leave unit (a) through gas outlet (a4), which is preferably located opposite to gas inlet (a1), most preferably at the top part of unit (a). Spent washing liquid in which the aromatic compounds are absorbed, is collected, typically at the bottom, and discharged through liquid outlet (a5).

The absorbing unit (a) may further comprise a condenser (a8) for removing water and traces of ammonia and HCl is present in the incoming gas. If present, condenser (a8) is located upstream of inlet (a1). Condenser (a8) preferably operates at a temperature of 2 - 15 °C, most preferably 3 - 7 °C. Safety filter (a7), if present, may for example be an aerosol filter or an electrostatic precipitator. The absorbing unit (a) may further comprise means (a3) for heating or cooling, typically for heating the gas, in particular the mixture of gas and washing liquid during contacting, such that absorption can be operated at temperatures in the range of 15 to 250 °C. Means (a3) may be located upstream of inlet (a1), i.e. in the connectivity between outlet (a'4) and inlet (a1), and downstream of condenser (a8), if present, such that the gas to be purified from monocyclic aromatic compounds is brought to the desired temperature prior to introduction in unit (a). Means (a3) typically take the form of a heater or heat exchanger as known in the art. It is preferred that unit (a) is designed to operate at atmospheric or slightly superatmospheric pressures.

The process may also comprise a module (a') or (a"), as defined further above. This module is preferably configured in the same way as module (a). Thus, everything described for module (a) herein and preferred embodiments thereof preferably equally applies to modules (a') as well as module (a"). Further, for module (a'), it is preferred that means (a'3) for heating or cooling, typically for heating the gas, in particular the mixture of gas and washing liquid during contacting, such that absorption can be operated at temperatures in the range of 15 - 60 °C, preferably 25 - 50 °C, more preferably 30 - 40 °C. Further, for module (a"), it is preferred that means (a"3) for heating or cooling, typically for heating the gas, in particular the mixture of gas and washing liquid during contacting, such that absorption can be operated at temperatures in the range of 60 - 250 °C, preferably 90 - 220 °C, more preferably 120 - 190 °C.

### Stripping unit (b)

The system comprises a stripping unit (b), wherein the aromatic compounds are desorbed from the washing liquid by a stripping gas. The stripping unit can be a tray tower, packed column, bubble column, spray tower or the like. The stripping gas can be co-currently or, preferably, counter-currently contacted with the spent polysiloxane. Unit (b) comprises a liquid inlet (b1) for receiving the spent washing liquid originating from unit (a). Inlet (b1) is in fluid connection with outlet (a5). The connectivity between outlet (a5) and inlet (b1) may contain a pump (b5), a safety filter (b6) and a heater (b7). Unit (b) further comprises a gas inlet (b2) for receiving a stripping gas. In a preferred configuration of unit (b), inlets (b1) and (b2) are located at opposite parts of the unit (b), preferably liquid inlet (b1) is located in the top part of the unit (b) and gas inlet (b2) is located in the bottom part of unit (b). The loaded stripping gas, which comprises the aromatic compounds, may be discharged via gas outlet (b3). Unit (b) further comprises a liquid outlet (b4) for discharging a stripped washing liquid, which is preferably returned to absorption unit (a) through fluid connection between outlet (b4) and inlet (a2), optionally through a pump (b8) and a cooler (b9), to enable reuse of the washing liquid in unit (a). Heater (b7) and cooler (b9) are advantageously in heat exchanging communication. Heater (b7) is preferably capable of raising the temperature of the spent washing liquid at least 50 °C, preferably in the range of 80 °C to 120 °C, with respect to the temperature in unit (a). Instead of or in addition to heater (b7) and cooler (b9), depressurising and pressurising devices can be inserted.

Stripping unit (b) is preferably designed to operate at a temperature between 70 and 120 °C above the temperature of the absorption column, more preferably about 100 °C above the temperature of the absorption column. Stripper (b) preferably is capable of operating at a temperature in the range of 120 to 250 °C. Heater (b7) is preferably capable of raising the temperature of the spent washing liquid to such a temperature. In a preferred embodiment, unit (b) is designed to operate at reduced pressure compared to unit (a).

The process may also comprise a module (b') or (b"), as defined further above. This module is preferably configured in the same way as module (b). Thus, everything described for module (b) herein and preferred embodiments thereof preferably equally applies to modules (b') as well as module (b").

### Separation module (c)

The loaded stripping gas issuing from stripping unit (b) through outlet (b3) is advantageously led to separation module (c). Separation module (c) comprises an inlet (c1) for receiving the loaded stripping gas from module (b). Inlet (c1) is in fluid connection with outlet (b3). The connectivity between outlet (b3) and inlet (c1) may contain a pump (c8). The loaded stripping gas is received to means (c2) for separating the aromatic compounds from the loaded stripping gas by condensing steam and/or aromatic compounds. In a preferred embodiment, means (c2) include a condenser, wherein at least one of the aromatic compounds and the steam of the stripping gas are capable of condensing, preferably by cooling. Condenser (c2) preferably is a cooler optionally in combination with a pressurising device, and preferably is capable of operating at a temperature of 4 - 40 °C, preferably 5 - 30 °C. Preferably, means (c2) in the form of a condenser is capable of condensing both the aromatic compounds and the steam comprised in the stripping gas. The skilled person understand how to design condenser (c2) in order to condense the aromatic compounds and the stripping gas, depending on the nature of the stripping gas. According to this embodiment, it is preferred that the stripping gas is steam, such that condensation of substantially all of the stripping gas in condenser (c2) is easily accomplished. Module (c) further comprises an outlet (c3) for discharging the cleared, typically condensed stripping gas, and an outlet (c4) for discharging the aromatic compounds. Outlet (c3) is preferably in fluid connection with inlet (b2), optionally via a mass flow controller a heater (c7) that is capable of vaporizing the stripping gas if discharged in condensed form from outlet (c3), to enable reuse of the stripping gas.

Module (c) preferably further comprises a collection vessel (c5), wherein the condensed liquids emerging from condenser (c2) are collected. The collection vessel (c5) is equipped with a liquid outlet (c4) for discharging aromatic compounds and a liquid outlet (c3) for discharging the condensed stripping gas. Vessel (c5) is advantageously equipped with means for liquid-liquid separation, as known in the art. Such separation is capable of separating a two-layer liquid system comprising an aqueous layer of condensed stripping gas and an organic layer of condensed aromatic compounds. Such separation is conveniently based on refractive index. Herein, outlet (c3) is preferably in fluid connection with inlet (b2) of stripping unit (b), preferably via a heater (c7) that is capable of vaporizing the condensed stripping gas to enable reuse of the stripping gas in unit (b).

The loaded stripping gas issuing from stripping unit (b) through outlet (b3) may contain a small amount of the gas to be purified (e.g. CH₄, CO, CO₂), which remains gaseous in condenser (c2), i.e. permanent gases. These are preferably discharged via a gas outlet (c6) which is in fluid connection with inlet (a1) of unit (a). Such recycle of permanent gases increases the yield of the purified (energy) gas that is obtainable by the process according to the invention, and the maximal amount of valuable aromatic compounds is extracted from the incoming gas. At the same time contamination of the stripping gas with permanent gases is prevented, which may thus be used longer (i.e. more cycles) before the need of cleaning or discarding and replacing thereof. Any remaining aromatic compounds that are not condensed in condenser (c2) are subjected again to absorption in unit (a).

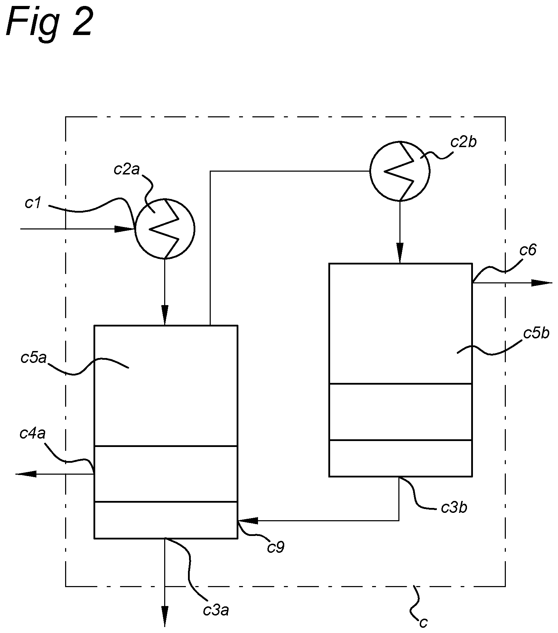

A preferred configuration of module (c) is depicted in figure 2. Herein, means (c2) contain two condensers (c2a) and (c2b), both equipped with a collection vessel (c5a) and (c5b). Loaded stripping gas entering module (c) via inlet (c1) is led to a first condenser (c2a), which is capable of operating at a temperature of 15 - 35 °C, preferably at ambient temperature. Employing such temperatures reduces the operating costs associated with cooling the bulk of loaded stripping gas that is fed to module (c), while at the same time enables condensation of the majority of the aromatic compounds comprised in the loaded stripping gas. The condensed liquids emerging from condenser (c2a) are collected in collection vessel (c5a), which is advantageously equipped with means for liquid-liquid separation. The remainder of the loaded stripping gas, i.e. which is not condensed in the first condenser (c2a), is led to a second condenser (c2b), which is capable of operating at a temperature of 4 - 10 °C, preferably 4 - 5 °C. At such operation temperatures, the remaining components of the loaded stripping gas are capable of condensing, except for the permanent gases dissolved in the loaded stripping gas. As only a minor part of the loaded stripping gas is fed to condenser (c2b), the operating costs for cooling are kept as low as possible, while efficient collection of substantially all of the aromatic compounds that are stripped from the washing liquid is readily accomplished. The permanent gases are discharged from module (c) via outlet (c6), while the condensate is collected in collection vessel (c5b), which is advantageously equipped with means for liquid-liquid separation. The immiscible compositions that are collected in vessels (c5a) and (c5b) may be combined, preferably in one of the collection vessels, which is equipped with means for liquid-liquid separation, while the other collection vessel contains a liquid outlet for discharging the immiscible composition the prior collection vessel. The collection vessel equipped with means for liquid-liquid separation then contains a liquid inlet for receiving the immiscible composition from the other collection vessel, and a liquid outlet for discharging condensed aromatic compounds and a liquid outlet for discharging the condensed stripping gas. Conveniently, collection vessel (c5a) is equipped with means for liquid-liquid separation, and contains a liquid inlet (c9) for receiving the immiscible composition from the collection vessel, and a liquid outlet (c4a) for discharging condensed aromatic compounds and a liquid outlet (c3a) for discharging the condensed stripping gas. In this embodiment, collection vessel (c5b) contains a liquid outlet (c3b) for discharging an immiscible composition of condensed aromatic compounds and condensed stripping gas. Herein, outlet (c3b) is in fluid connection with inlet (c9). In an alternative embodiment, collection vessel (c5b) further comprises a liquid outlet (c4b) for discharging condensed aromatic compounds while liquid outlet (c3b) is used for discharging condensed stripping gas to collection vessel (c5a). As such, all condensed stripping gas is collected in a single collection vessel, from which it is discharged and preferentially returned to module (b). Thus, outlet (c3a) is preferably in fluid connection with inlet (b2) of stripping unit (b), preferably via a mass flow controller and a heater (c7) that is capable of vaporizing the condensed stripping gas to enable reuse of the stripping gas in unit (b). A single fraction of aromatic compounds is discharged via outlet (c4a), or two fractions of aromatic compounds are discharged via outlets (c4a) and (c4b) respectively, which may or may not be combined afterwards.

The process may also comprise a module (c') or (c"), as defined further above. This module is preferably configured in the same way as module (c). Thus, everything described for module (c) herein and preferred embodiments thereof preferably equally applies to modules (c') as well as module (c").

### Optional further modules

The system may further comprise a module (e) for gasifying or pyrolizing coal, biomass or a combination thereof, preferably biomass, such as (organic) waste. Thus, module (e) is a gasifier (gasification reactor) or pyrolyser (pyrolysis reactor). Such reactors are known in the art. Typically, a flow of biomass and/or coal is introduced into module (e) through an inlet (e1). The gasifying gas can be e.g. air, oxygen and/or steam, which introduced through the same inlet (e1) or a further inlet. The biomass and/or coal can be further supplied with tar-like components issuing from tar stripping unit (b') via outlet (b'3), i.e. outlet (b'3) and an inlet of module (e), e.g. inlet (e1) or a further inlet, are preferably in fluid connection. As such, the tar-like components are recycled and subjected to a further gasification or pyrolysis process, and thus converted in further valuable compounds such as energy gas components and/or aromatic compounds. In case module (e) is a gasifier, it is preferably capable of gasifying biomass at a temperature in the range of 600 - 1300 °C using sub-stoichiometric quantities of oxygen. In case module (e) is a pyrolyser, it is preferably capable of pyrolysing biomass at the same or somewhat lower temperature (e.g. from 450 °C up to 950 °C). As alternative to the embodiment wherein the tar-like components are recycled to reactor (e) itself, the tar-like components may also be used as fuel for heating the reactor (e) to the desired temperature and/or maintaining the reactor (e) at the desired temperature.

Gas issuing from module (e) may be fed to a particulate clearing module (f), wherein particulate material such as dust is removed. Such clearing is preferably based on gravitation and more particularly with a cyclone. Next, the gas, optionally cleared from particulate material, may be treated in a pre-cleaning module (g), especially when the gas contains substantial levels of heavy tar-like components, i.e. having more than 18 ring atoms (i.e. boiling points above about 450 °C at 1 bar), in particular if it contains components of more than 24 ring atoms (i.e. boiling points above about 520 °C at 1 bar). Part or most of these heavy tar-like components may be caught in module (g), such that the gas issuing from said module is depleted in such heavy tar-like components. Besides tars based on hydrocarbons and dust, also sulphur and chloridecontaining material can be removed from the gas flow. Module (g) typically employs an aromatic hydrocarbon based liquid to cool the gas and condense tar-like components, which are collected and subsequently separated from the hydrocarbon liquid. Module (g) preferably operates at a higher temperature than unit (a'), for example a liquid inlet temperature of between 150 and 300 °C, and a gas inlet temperature between 250 °C and 900 °C. In an optional next step, the (partly cleaned) gas may pass through a filter (h), for example an electrostatic precipitator, which removes dust. Instead of scrubber (g) and/or filter (h), an alternative tar and dust removing step may be inserted, such as an aerosol scavenger. Then, the gas can be entered into the system of the invention through inlet (a1) of unit (a) or optionally through inlet (a'1) of unit (a').

The optional preceding modules (e), (f), (g) and (h) are only schematically depicted in the accompanying figure. More details are given in WO 2008/010717 and WO 2011/037463. Depending on the quality of the input gas, one or more or all of the intermittent cleaning steps and modules described above can be dispensed with.

### Figures

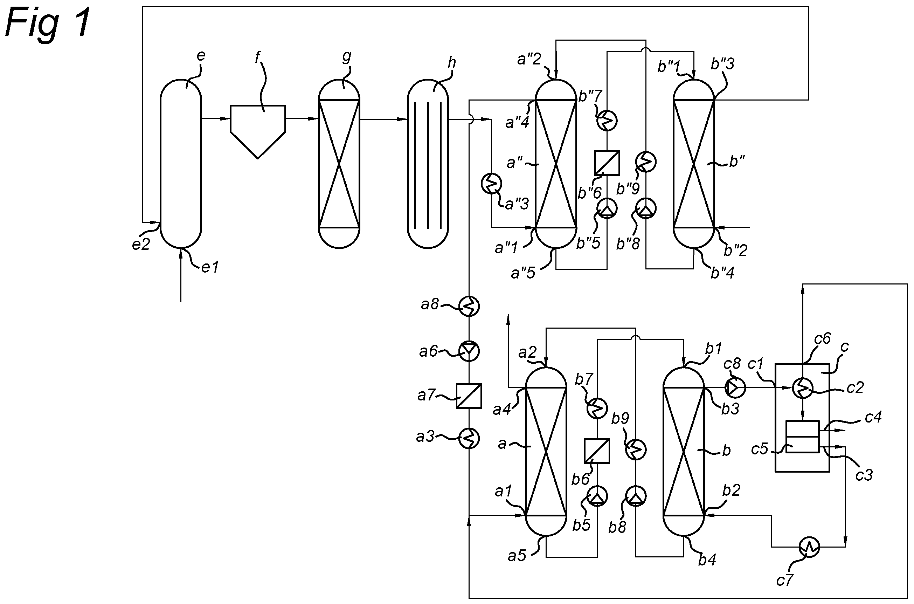

Figure 1 depicts a preferred embodiment of the process and system with reference to the description of the system above and accompanying reference numbers.
Figure 2 depicts a preferred embodiment of separation module (c), with reference to the description of the system above and accompanying reference numbers.

### Examples

### Example 1

Beech wood (5 kg/h) was subjected to gasification with 1 kg steam per h in an indirect allothermal biomass gasifier (MILENA), which is coupled to a pre-washing unit (OLGA absorber). A trace amount of argon, which was used as tracker, was added to the gasifier gas, which was fed to the pre-washing unit with an average gas flow of about 15 NI/min, based on dry gas. The partly cleaned gas was led via a cooler (T = 5 °C), a safety filter (soxhlet filter) and glass beads to an absorber (BTEX scrubber). The absorber of the BTEX scrubber operated at 35 °C and ambient pressure with polysiloxane as a washing liquid. The loaded absorbent was stripped at 120 °C using 205 - 820 g/h steam. Loaded stripping gas was led to condensers via tubes heated at 120 - 160 °C. The first condenser operated at 25 - 27 °C and the second condenser at 4 - 5 °C. Liquids were collected from the first condenser, while remaining gases were led to the second condenser. Liquids were collected from the second condenser. The scrubbed gas was subjected to HDS and subsequently steam reforming to obtain a bio-SNG. The experiment was ran continuously for 75 h at ambient pressure.

Collection of liquids occurred by first collecting the aqueous layer by opening a tap at the bottom of the collection flask. Collection of the aqueous layer was stopped just prior to the meniscus reached the tap. An as small as possible mixed fraction was collected and discarded, after which the organic layer was completely drained in a separate flask. A total of 1.17 kg of organic layer was collected (885 g from the first condenser at 26 °C and 285 g from the second condenser at 5 °C) over the complete duration of the experiment, of which 86.6 wt% benzene, 6.5 wt% toluene and 0.20 wt% xylene. A detailed compositional analysis of the combined organic layers is given in Table 1. The average compositions of the gas flows over the complete duration of the experiment, as determined by micro-GC, are given in Table 2.

**Table 1: Composition of the combined organic layers**

| Compound | First condenser (26 °C) | Second condenser (4 - 5 °C) | Total |
|---|---|---|---|
| total (g) | 884.5 | 284.7 | 1169.2 |
| total BTEX (g) | 826.2 | 266.9 | 1093.0 |
| benzene (g, wt%) | 753.6 (85.2 wt%) | 258.9 (90.9 wt%) | 1012.5 (86.6 wt%) |
| toluene (g, wt%) | 68.7 (7.77 wt%) | 7.77 (2.73 wt%) | 76.50 (6.54 wt%) |
| xylene (g, wt%) | 2.26 (0.26 wt%) | 0.06 (0.02 wt%) | 2.32 (0.20 wt%) |
| ethylbenzene (g, wt%) | 1.66 (0.19 wt%) | 0.07 (0.02 wt%) | 1.74 (0.15 wt%) |
| styrene (wt%) | 1.48 | 0.07 | 1.14 |
| cresol (wt%) | 0.37 | 0.00 | 0.28 |
| naphthalene (wt%) | 0.75 | 0.00 | 0.57 |
| further aromatic compounds (wt%) | 0.82 | 0.39 | 0.72 |
| thiophene (wt%) | 0.12 | 0.13 | 0.12 |
| water (wt%) | 0.07 | 0.67 | 0.22 |

**Table 2: Composition of the gas flows (based on dry weight)**

| Component | Gasifier gas ^{[a]} | pre-washed gas ^{[b]} | purified gas ^{[c]} |
|---|---|---|---|
| inert (vol%) ^{[d]} | 3.7 | 4.8 | 5.4 |
| CH₄ (vol%) | 11.3 | 10.9 | 10.5 |
| CO (vol%) | 32.0 | 28.9 | 23.7 |
| CO₂ (vol%) | 25.3 | 25.0 | 27.1 |
| C₂ (vol%) ^{[e]} | 3.9 | 3.9 | 3.0 |
| H₂S (ppmV) | 100 | 151 | 88 |
| COS (ppmV) | 3 | 5 | 0 |
| benzene (ppmV) | 8131 | 4680 | 271 |
| toluene (ppmV) | 596 | 261 | 0 |
| thiophene (ppmV) | 20 | 17 | 0.9 |
| tar (mg/Nm³) | 17565 | 680 | 61 |
| H₂ (vol%) ^{[f]} | 22.9 | 26.0 | 30.0 |

[a] gas emerging from the gasifier, prior to being subjected to pre-washing; [b] gas emerging from the OLGA absorber, prior to being subjected to the BTEX scrubber; [c] gas emerging from the BTEX scrubber; [d] Ar + N₂; [e] ethane + ethylene + acetylene; [f] H₂ content estimated, based on total volume of 100 vol%.

Tar-like components were mainly removed in the pre-washing step, while monocyclic aromatic compounds, such as benzene, toluene, ethylbenzene and even thiophene, were largely maintained in the permanent gas stream. The BTEX scrubber effectively removed the monocyclic aromatic components. The benzene concentration in the partly cleaned gas emerging from the OLGA absorber was between 4000 and 7000 ppm (vol.), which was lowered to ~ 300 ppm (vol.) in the gas stream emerging from the BTEX-scrubber. The average removal of benzene amounted to 95 % using a steam flow of 820 g/h, which reduced to 89 % and 87 % at a gas flow of 410 g/h and 205 g/h respectively. 100 % of the toluene was removed at all gas flows. Only trace amounts of tar-like components were obtained in the organic layers obtained in the first and second condensers.

The composition of the gas was analyzed prior to and after the condensers, and the removal percentages obtained during the BTEX scrub of a variety of compounds are given in Table 3. The aromatic compounds benzene, toluene, xylene and thiophene (and its derivatives) were effectively removed during the BTEX scrub, wherein generally the highest steam flow provided the highest removal. At the same time, permanent gases such as C₁-C₃ hydrocarbons (alkanes and alkenes) are effectively retained in the gas stream. Especially methane, CO and CO₂ are completely retained. Any transport thereof to the stripping gas is cancelled when the permanent gases are recycled to the entrance of the BTEX scrubber. The content of the permanent gases in the fuel gas (at the entrance of the BTEX scrubber) and in the permanent gas stream after stripping (downstream of the second condenser) are given in Table 4. In view of its very small volume, nitrogen gas was added to the permanent gas stream to enable measure of its contents. The amount of these permanent gases that were transported to the stripping gas is also included in Table 4. The permanent gas stream further contained 4.6 vol% benzene (based on the permanent gas stream without added nitrogen).

**Table 3: Removal percentages for certain compounds**

| Compound | | steam flow during stripping | | |
|---|---|---|---|---|
| | | 205 g/h | 410 g/h | 820 g/h |
| Aromatic compounds | | | | |
| | benzene | 87 % | 89 % | 95 % |
| | toluene | 93 % | 92 % | 100 % |
| | xylene | 100 % | 100 % | 100 % |
| Sulphur components | | | | |
| | thiophene | 94 % | 91 % | 96 % |
| | 2-methyl-thiophene ^{[b]} | 100 % | 100 % | 100 % |
| | 3-methyl-thiophene ^{[b]} | 100 % | 100 % | 100 % |
| | COS | 6 % | 0 % | 14 % |
| | methyl mercaptan | 67 % | 68 % | 60 % |
| | ethyl mercaptan | 76 % | 78 % | 77 % |

| | | | | |
|---|---|---|---|---|
| [a] nd = not determined [b] no methyl-thiophenes were detected after the BTEX-scrub. | | | | |

**Table 4: Experimentally determined transport values of permanent gases**

| Permanent gases | In fuel gas | In strip gas | transported |
|---|---|---|---|
| methane (vol%) | 11.00 | 0.13 | 1.18 |
| ethane (vol%) | 0.20 | 0.0094 | 4.70 |
| ethene (vol%) | 3.10 | 0.168 | 5.43 |
| ethyne (vol%) | 0.155 | 0.0185 | 11.94 |
| CO₂ (vol%) | 25.00 | 0.72 | 2.88 |
| CO (vol%) | 28.00 | 0.07 | 0.25 |
| H₂ (vol%) | 26.00 | 0.00 | 0.00 |
| H₂S (ppmV) | 150.00 | 63 | 42.00 |
| COS (ppmV) | 5.00 | 0.00 | 0.00 |

The results in Table 3 show that the BTEX-scrub, i.e. step (a) of the process according to the invention, effectively removes a fraction of aromatic compounds, that contains almost exclusively BTEX. The BTEX fraction can be used as deemed fit, e.g. marketed as bio-based BTEX or the like and the energy gas is sufficiently purified from tar-like components and aromatic components by virtue of the combined pre-washing and BTEX-scrub such that conversion into bio-SNG is readily accomplished.

### Example 2

Refuse-derived fuel (RDF) (3.9 kg/h) was subjected to gasification with 2 kg steam per h in an indirect allothermal biomass gasifier (MILENA), which is coupled to a pre-washing unit (OLGA absorber). Nitrogen gas was added to the steam flow to maintain sufficient gas flow throughout the system. The gasifier gas, which was fed to the pre-washing unit with an average gas flow of about 15 NI/min, based on dry gas. The partly cleaned gas was led via a cooler (T = 5 °C), a safety filter (glass beads) and a pre-washing unit to remove tars (washing liquid = polymethylphenylsiloxane; *T* = 80 °C, ambient pressure) to an absorber (BTEX scrubber). The absorber of the BTEX scrubber operated at 35 °C and ambient pressure with polymethylphenylsiloxane as a washing liquid. The loaded absorbent was stripped at 160 °C using 0.25 m³/h steam. Loaded stripping gas was led to condensers via tubes heated at 160 °C. The first condenser operated at 25 - 27 °C and the second condenser at 4 - 5 °C. Liquids were collected from the first condenser, while remaining gases were led to the second condenser. Liquids were collected from the second condenser. A fraction of monocyclic aromatic compounds comprising benzene, toluene and xylene was collected. The experiment was ran continuously for 3.5 h at ambient pressure. The average compositions of the gas flows over the complete duration of the experiment, as determined by micro-GC, are given in Table 5.

**Table 5: Composition of the gas flows**

| Component | Gasifier gas ^{[a]} | pre-washed gas ^{[b]} | purified gas ^{[c]} |
|---|---|---|---|
| inert (vol%) ^{[d]} | 51.7 | 53.8 | 53.4 |
| CH₄ (vol%) | 7.8 | 7.7 | 7.5 |
| CO (vol%) | 9.0 | 9.0 | 9.0 |
| CO₂ (vol%) | 11.4 | 12.2 | 11.2 |
| C₂ (vol%) ^{[e]} | 7.4 | 7.8 | 7.2 |
| H₂S (ppmV) | 524 | 633 | 449 |
| COS (ppmV) | 9 | 16 | 0 |
| benzene (ppmV) | 9853 | 9135 | 302 |
| toluene (ppmV) | 1490 | 1269 | 35 |
| thiophene (ppmV) | 43 | 29 | nd ^{[f]} |
| tar (mg/Nm³) | 38373 | 809 | 61 |
| H₂ (vol%) ^{[g]} | 8.4 | 8.4 | 8.4 |

| | | | |
|---|---|---|---|
| [a] gas emerging from the gasifier, prior to being subjected to pre-washing; [b] gas emerging from the OLGA absorber, prior to being subjected to the BTEX scrubber; [c] gas emerging from the BTEX scrubber; [d] Ar + N₂; [e] ethane + ethylene + acetylene; [f] not determined; [g] H₂ content estimated, based on total volume of 100 vol%. | | | |

## Claims

1. Process for isolating a fraction of monocyclic aromatic compounds from a gas originating from the gasification of biomass, waste or a combination thereof, comprising:
(a) contacting the gas with a washing liquid, at a temperature of 15 - 60 °C, to obtain the purified gas and a spent washing liquid comprising monocyclic aromatic compounds;
(b) contacting the spent washing liquid with a stripping gas comprising at least 50 vol.% steam, to obtain a loaded stripping gas comprising monocyclic aromatic compounds and a stripped washing liquid; and
(c) separating the monocyclic aromatic compounds from the loaded stripping gas obtained in step (b) by condensation of the steam and the monocyclic aromatic compounds comprised in the loaded stripping gas to obtain an immiscible liquid composition and isolating the monocyclic aromatic compounds therefrom,
wherein the monocyclic aromatic compounds include benzene, toluene, ethylbenzene and xylene.

2. Process according to claim 1, wherein the gas further comprises tar-like components, wherein tar-like components are aromatic compounds having a boiling point above 200 º C at atmospheric pressure, which are removed from the gas in a step (a') that is performed prior to step (a).

3. Process according to claim 1 or 2, wherein the gas is subjected to a pre-washing step prior to being subjected to step (a) the pre-washing comprises:
(a") contacting the gas with a pre-washing liquid, at a temperature of 60 - 250 °C, to obtain pre-purified gas which is subjected to step (a) and a spent pre-washing liquid comprising aromatic compounds including tar-like components;
(b") contacting the spent pre-washing liquid with a pre-stripping gas, to obtain a loaded pre-stripping gas comprising the tar-like components and a stripped pre-washing liquid; and
(c") optionally separating the tar-like components from the loaded pre-stripping gas.

4. Process according to claim 3, wherein the pre-washing liquid comprises at least 50 vol.% steam and the separating of step (c") is performed by condensation of the steam and the aromatic compounds comprised in the loaded pre-stripping gas to obtain an immiscible composition and isolating the aromatic compounds therefrom.

5. Process according to any one of the preceding claims, wherein in step (c) and optionally (c") the aromatic compounds are isolated from the immiscible liquid by oil/water separation.

6. Process according to any one of the preceding claims, wherein condensation in step (c) and optionally (c") is accomplished by lowering the temperature and/or increasing the pressure of the loaded stripping gas.

7. Process according to any one of the preceding claims, wherein the gas comprises the aromatic compounds and one or more components selected from H₂, CO and C₁₋₄ alkanes, preferably wherein the gas is an energy gas, which preferably originates from the gasification of biomass, waste, coal or a combination thereof.

8. Process according to any one of the preceding claims, wherein the washing liquid comprises an organic polysiloxane.

9. Process according to any one of the preceding claims, wherein the stripping gas comprises at least 95 vol.% steam.

10. Process according to any one of the preceding claims, wherein step (b) is performed at a temperature of 120 - 220 °C.

11. Process according to any one of the preceding claims, further comprising removing sulphur-containing components from the gas by hydrodesulphurisation or adsorption, preferably by adsorption.

## Patentansprüche

1. Verfahren zur Isolierung einer Fraktion von monozyklischen aromatischen Verbindungen aus einem Gas, das aus der Vergasung von Biomasse, Abfall oder einer Kombination davon stammt, umfassend:
(a) Inkontaktbringen des Gases mit einer Waschflüssigkeit bei einer Temperatur von 15 - 60 °C, um das gereinigte Gas und eine verbrauchte Waschflüssigkeit, die monozyklische aromatische Verbindungen umfasst, zu erhalten;
(b) Inkontaktbringen der verbrauchten Waschflüssigkeit mit einem Strippgas, das mindestens 50 Vol.-% Wasserdampf umfasst, um ein beladenes Strippgas, das monocyclische aromatische Verbindungen umfasst, und eine gestrippte Waschflüssigkeit zu erhalten; und
(c) Abtrennen der monocyclischen aromatischen Verbindungen aus dem beladenen Strippgas, das in Schritt (b) erhalten wurde, durch Kondensation des Wasserdampfs und der monocyclischen aromatischen Verbindungen, die in dem beladenen Strippgas umfasst sind, um eine nicht mischbare flüssige Zusammensetzung zu erhalten, und Isolieren der monocyclischen aromatischen Verbindungen daraus,
wobei die monocyclischen aromatischen Verbindungen Benzol, Toluol, Ethylbenzol und Xylol beinhalten.

2. Verfahren nach Anspruch 1, wobei das Gas ferner teerartige Komponenten umfasst, wobei teerartigen Komponenten aromatische Verbindungen sind, die einen Siedepunkt über 200 °C bei Atmosphärendruck aufweisen, die aus dem Gas in einem Schritt (a') entfernt werden, der vor Schritt (a) durchgeführt wird.

3. Verfahren nach Anspruch 1 oder 2, wobei das Gas einem Vorwaschschritt unterzogen wird, bevor es Schritt (a) unterzogen wird, wobei das Vorwaschen umfasst:
(a") Inkontaktbringen des Gases mit einer Vorwaschflüssigkeit bei einer Temperatur von 60 - 250 °C, um vorgereinigtes Gas zu erhalten, das Schritt (a) unterzogen wird, und eine verbrauchte Vorwaschflüssigkeit, die aromatische Verbindungen, die teerartige Komponenten beinhalten, umfasst;
(b") Inkontaktbringen der verbrauchten Vorwaschflüssigkeit mit einem Vorstrippgas, um ein beladenes Vorstrippgas zu erhalten, das die teerartigen Komponenten umfasst, und eine gestrippte Vorwaschflüssigkeit; und
(c") optionales Abtrennen der teerartigen Komponenten aus dem beladenen Vorstrippgas.

4. Verfahren nach Anspruch 3, wobei die Vorwaschflüssigkeit mindestens 50 Vol.-% Wasserdampf umfasst und das Abtrennen in Schritt (c") durch Kondensation des Wasserdampfs und der aromatischen Verbindungen, die in dem beladenen Vorstrippgas umfasst sind, durchgeführt wird, um eine nicht mischbare Zusammensetzung zu erhalten, und die aromatischen Verbindungen daraus isoliert werden.

5. Verfahren nach einem der vorstehenden Ansprüche, wobei in Schritt (c) und optional (c") die aromatischen Verbindungen durch Öl-Wasser-Trennung aus der nicht mischbaren Flüssigkeit isoliert werden.

6. Verfahren nach einem der vorstehenden Ansprüche, wobei die Kondensation in Schritt (c) und optional (c") durch Absenken der Temperatur und/oder Erhöhen des Drucks des beladenen Strippgases erreicht wird.

7. Verfahren nach einem der vorstehenden Ansprüche, wobei das Gas die aromatischen Verbindungen und eine oder mehrere Komponenten umfasst, ausgewählt aus H2, CO und C-Alkanen, bevorzugt, wobei das Gas ein Energiegas ist, das bevorzugt aus der Vergasung von Biomasse, Abfällen, Kohle oder einer Kombination davon stammt.

8. Verfahren nach einem der vorstehenden Ansprüche, wobei die Waschflüssigkeit ein organisches Polysiloxan umfasst.

9. Verfahren nach einem der vorstehenden Ansprüche, wobei das Strippgas mindestens 95 Vol.-% Wasserdampf umfasst.

10. Verfahren nach einem der vorstehenden Ansprüche, wobei Schritt (b) bei einer Temperatur von 120 - 220 °C durchgeführt wird.

11. Verfahren nach einem der vorstehenden Ansprüche, das ferner Entfernen von schwefelenthaltenden Komponenten aus dem Gas durch Hydrodesulfurierung oder Adsorption, bevorzugt durch Adsorption, umfasst.

## Revendications

1. Procédé pour isoler une fraction de composés aromatiques monocycliques à partir d'un gaz provenant de la gazéification de biomasse, de déchets ou d'une combinaison de ceux-ci, comprenant :
(a) mettre le gaz en contact avec un liquide de lavage, à une température de 15 à 60 °C, pour obtenir le gaz purifié et un liquide de lavage usé comprenant des composés aromatiques monocycliques ;
(b) mettre le liquide de lavage usé en contact avec un gaz de stripage comprenant au moins 50 % en volume de vapeur, pour obtenir un gaz de stripage chargé comprenant des composés aromatiques monocycliques et un liquide de lavage strippé ; et
(c) séparer les composés aromatiques monocycliques du gaz de stripage chargé obtenu à l'étape (b) par condensation de la vapeur et des composés aromatiques monocycliques compris dans le gaz de stripage chargé pour obtenir une composition liquide non miscible et par isolement des composés aromatiques monocycliques de celle-ci,
où les composés aromatiques monocycliques incluent le benzène, le toluène, l'éthylbenzène et le xylène.

2. Procédé selon la revendication 1, où le à gaz comprend en outre des composants de type goudron, où les composants de type goudron sont des composés aromatiques ayant un point d'ébullition supérieur à 200 °C à pression atmosphérique, qui sont éliminés du gaz dans une étape (a') effectuée avant l'étape (a).

3. Procédé selon la revendication 1 ou 2, où le gaz est soumis à une étape de prélavage avant d'être soumis à l'étape (a), le prélavage comprenant :
(a") mettre le gaz en contact avec un liquide de prélavage, à une température de 60 à 250 °C, pour obtenir un gaz pré-purifié qui est soumis à l'étape (a) et un liquide de prélavage usé comprenant des composés aromatiques incluant des composants de type goudron ;
(b") mettre le liquide de prélavage usé en contact avec un gaz de pré-stripage, pour obtenir un gaz de pré-stripage chargé comprenant les composants de type goudron et un liquide de lavage strippé ; et
(c") de manière optionnelle, séparer les composants de type goudron du gaz de pré-stripage chargé.

4. Procédé selon la revendication 3, où le liquide de prélavage comprend au moins 50 % en volume de vapeur et la séparation mentionnée à l'étape (c") est effectuée par condensation de la vapeur et des composés aromatiques compris dans le gaz de pré-stripage chargé pour obtenir une composition non miscible et par isolement des composés aromatiques de celle-ci.

5. Procédé selon l'une quelconque des revendications précédentes, où dans l'étape (c) et de manière optionnelle (c"), les composés aromatiques sont isolés du liquide non miscible par séparation huile/eau.

6. Procédé selon l'une quelconque des revendications précédentes, où la condensation à l'étape (c) et de manière optionnelle (c") est réalisée en abaissant la température et/ou en augmentant la pression du gaz de stripage chargé.

7. Procédé selon l'une quelconque des revendications précédentes, où le gaz comprend des composés aromatiques et un ou plusieurs composants choisis parmi H₂, CO et les alcanes C₁₋₄, où de préférence le gaz est un gaz d'énergie, qui provient de préférence de la gazéification de biomasse, de déchets, de charbon ou d'une combinaison de ceux-ci.

8. Procédé selon l'une quelconque des revendications précédentes, où le liquide de lavage comprend un polysiloxane organique.

9. Procédé selon l'une quelconque des revendications précédentes, où le gaz de stripage comprend au moins 95 % en volume de vapeur.

10. Procédé selon l'une quelconque des revendications précédentes, où l'étape (b) est effectuée à une température de 120 à 220 °C.

11. Procédé selon l'une quelconque des revendications précédentes, comprenant en outre l'élimination des composants contenant du soufre du gaz par hydrodésulfuration ou adsorption, de préférence par adsorption.
